# EUROPEAN PATENT APPLICATION

(11) **EP 3 378 949 A1**
(43) Date of publication of application: **26.09.2018**
(21) Application number: 17305327.3
(22) Date of filing: 22.03.2017
(51) Int. Cl.: C12Q 1/68

(54) **METHOD FOR DETERMINING THE POTENTIAL EFFICACY OF ANTICANCER TREATMENT**

(71) Applicant: Assistance Publique - Hôpitaux de Paris, 75004 Paris (FR); Institut National De La Recherche Agronomique, 75007 Paris (FR)
(72) Inventor: The designation of the inventor has not yet been filed
(74) Representative: Gevers & Orès

(57) **Abstract**

The present invention relates to methods for *ex vivo* determining whether a patient with metastatic melanoma is likely to benefit from a treatment with an anti CTLA-4 molecule, preferably ipilimumab, by analysing the gut microbiota in a fecal sample from said patient.

SEQUENCE LISTING

<110>  ASSISTANCE PUBLIQUE - HOPITAUX DE PARIS
       INSTITUT NATIONAL DE LA RECHERCHE AGRONOMIQUE

<120>  Method for determining the potential efficacy of anticancer
       treatment

<130>  CLBE/safl-F1020 63EP

<160>  32

<170>  PatentIn version 3.5

<210>  1
<211>  418
<212>  DNA
<213>  Parabacteroides distasonis

<400>  1
tgaggaatat tggtcaatgg ccgagaggct gaaccagcca agtcgcgtga gggatgaagg      60

ttctatggat cgtaaacctc ttttataagg gaataaagtg cgggacgtgt cccgttttgt     120

atgtacctta tgaataagga tcggctaact ccgtgccagc agccgcggta atacggagga     180

tccgagcgtt atccggattt attgggttta aagggtgcgt aggcggcctt ttaagtcagc     240

ggtgaaagtc tgtggctcaa ccatagaatt gccgttgaaa ctggggggct tgagtatgtt      300

tgaggcaggc ggaatgcgtg gtgtagcggt gaaatgcata gatatcacgc agaaccccga     360

ttgcgaaggc agcctgccaa gccattactg acgctgatgc acgaaagcgt gggggatc       418


<210>  2
<211>  402
<212>  DNA
<213>  uncultured bacterium NA48 AY975552

<400>  2
tggggaatat tgcacaatgg gggaaaccct gatgcagcga cgccgcgtga gtgaagaagt      60

atttcggtat gtaaagctct atcagcaggg aagaaagtga cggtacctga ataagaagcc     120

ccggctaact acgtgccagc agccgcggta atacgtaggg ggcaagcgtt atccggattt     180

actgggtgta aagggagcgt agacggcaag gcaagtctga agtgaaagcc cggtgcttaa     240

cgccgggact gctttggaaa ctgtttggct ggagtgccgg agaggtaagc ggaattccta     300

gtgtagcggt gaaatgcgta gatattagga agaacaccag tggcgaaggc ggcttactgg     360

acggtaactg acgttgaggc tcgaaagcgt ggggagcaaa ca                        402


<210>  3
<211>  396
<212>  DNA
<213>  butyrate producing bacterium SS2 1 AY305319

<400>  3
tggggaatat tgcacaatgg gggaaaccct gatgcagcga cgccgcgtga gtgaagaagt      60

atctcggtat gtaaagctct atcagcaggg aagaaaatga cggtacctga ctaagaagcc 120

ccggctaact acgtgccagc agccgcggta atacgtaggg ggcaagcgtt atccggaatt 180

actgggtgta aagggtgcgt aggtggtatg gcaagtcaga agtgaaaacc cagggcttaa 240

ctctgggact gcttttgaaa ctgtcagact ggagtgcagg agaggtaagc ggaattccta 300

gtgtagcggt gaaatgcgta gatattagga ggaacatcag tggcgaaggc ggcttactgg 360

actgaaactg acactgaggc acgaaagcgt ggggga 396

<210> 4
<211> 430
<212> DNA
<213> uncultured bacterium C3 13 GQ896957

<400> 4
tggggaatat tgggcaatgg gcgcaagcct gacccagcaa cgccgcgtga aggaagaagg 60

ctttcgggtt gtaaacttct tttgtcgggg acgagtagaa gacggtacct ggcgaataag 120

ccacggctaa ctacgtgcca gcagccgcgg taatacgtag gtggcaagcg ttgtccggat 180

ttactgggtg taaagggcgt gtagccggga aggcaagtca gatgtgaaat ccacgggctt 240

aactcgtgaa ctgcatttga aactactttt cttgagtatc ggagaggcaa tcggaattcc 300

tagtgtagcg gtgaaatgcg tagatattag gaggaacacc agtggcgaag gcggattgct 360

gggacgacaa ctgacgggtg agggcgcgaa agcgtggggg agcaaacagg gattagatac 420

cctgggtagt 430

<210> 5
<211> 401
<212> DNA
<213> Faecalibacterium prausnitzii

<400> 5
tggggaatat tgcacaatgg gggaaaccct gatgcagcga cgccgcgtgg aggaagaagg 60

tcttcggatt gtaaactcct gttgttgagg aagataatga cggtactcaa caaggaagtg 120

acggctaact acgtgccagc agccgcggta aaacgtaggt cacaagcgtt gtccggaatt 180

actgggtgta aagggagcgc aggcgggaga acaagttgga agtgaaatcc atgggctcaa 240

cccatgaact gctttcaaaa ctgtttttct tgagtagtgc agaggtaggc ggaattcccg 300

gtgtagcggt ggaatgcgta gatatcggga ggaacaccag tggcgaaggc ggcctactgg 360

gcaccaactg acgctgaggc tcgaaagtgt gggtagcaaa c 401

<210> 6
<211> 422
<212> DNA
<213> Gemmiger formicilis

<400>  6
tgggggatat tgcacaatgg gggaaaccct gatgcagcga cgccgcgtgg aggaagaagg      60

ttttcggatt gtaaactcct gtcgttaggg acgataatga cggtacctaa caagaaagca      120

ccggctaact acgtgccagc agccgcggta aaacgtaggg tgcaagcgtt gtccggaatt      180

actgggtgta aagggagcgc aggcggaccg gcaagttgga agtgaaaact atgggctcaa      240

cccataaatt gctttcaaaa ctgctggcct tgagtagtgc agaggtaggt ggaattcccg      300

gtgtagcggt ggaatgcgta gatatcggga ggaacaccag tggcgaaggc gacctactgg      360

gcaccaactg acgctgaggc tcgaaagcat gggtagcaaa caggattaga taccctggta      420

gt                                                                      422

<210>  7
<211>  398
<212>  DNA
<213>  uncultured bacterium RL246 aai74e12 DQ793623

<400>  7
tggggaatat tgcacaatgg gggaaaccct gatgcagcga cgccgcgtgg aggaagaagg      60

tcttcggatt gtaaactcct gttgttgggg aagataatga cggtacccaa caaggaagtg      120

acggctaact acgtgccagc agccgcggta aaacgtgggt cacaagcgtt gtccggaatt      180

actgggtgta aagggagcgc aggcgggaag acaagttgga agtgaaatct atgggctcaa      240

cccataaact gctttcaaaa ctgtttttct tgagtagtgc agaggtaggc ggaattcccg      300

gtgtagcggt ggaatgcgta gatatcggga ggaacaccag tggcgaaggc ggcctactgg      360

ggcaccaact gacgctgagg gctcgaaagt gtggggta                              398

<210>  8
<211>  204
<212>  DNA
<213>  Bacteroides ovatus

<400>  8
acgggaggca gcagtgagga atattggtca atgggcgaga gcctgaacca gccaagtagc      60

gtgaaggatg aaggctctat gggtcgtaaa cttcttttat atgggaataa agttttccac      120

gtgtggaatt ttgtatgtac catatgaata aggatcggct aactccgtgc cagcagccgc      180

ggtaatacgg aggatccgag cgtt                                              204

<210>  9
<211>  202
<212>  DNA
<213>  Faecalibacterium prausnitzii

<400>  9
acgggaggca gcagtgggga atattgcaca atgggggaaa ccctgatgca gcgacgccgc      60

gtggaggaag aaggtcttcg gattgtaaac tcctgttgtt gaggaagata atgacggtac      120

tcaacaagga agtgacggct aactacgtgc cagcagccgc ggtaaaacgt aggtcacaag 180

cgttgtccgg aattactggg tg 202

<210> 10
<211> 202
<212> DNA
<213> Eisenbergiella massiliensis

<400> 10
acgggaggca gcagtgggga atattgcaca atgggggaaa ccctgatgca gcgacgccgc 60

gtgagtgaag aagtatttcg gtatgtaaag ctctatcagc agggaagaaa atgacggtac 120

ctgactaaga agccccggct aactacgtgc cagcagccgc ggtaatacgt agggggcaag 180

cgttatccgg atttactggg tg 202

<210> 11
<211> 204
<212> DNA
<213> uncultured bacterium REC1M 21 AY343160

<400> 11
acgggaggca gcagtgggga atattgggca atgggcgcaa gcctgaccca gcaacgccgc 60

gtgaaggaag aaggctttcg ggttgtaaac ttcttttgtc agggacgagt agaagacggt 120

acctgacgaa taagccacgg ctaactacgt gccagcagcc gcggtaatac gtaggtggca 180

agcgttgtcc ggatttactg ggtg 204

<210> 12
<211> 402
<212> DNA
<213> butyrate producing bacterium L2 21 AJ270477

<400> 12
tggggaatat tgcacaatgg gcgaaagcct gatgcagcga cgccgcgtga gcgaagaagt 60

atttcggtat gtaaagctct atcagcaggg aagataatga cggtacctga ctaagaagca 120

ccggctaaat acgtgccagc agccgcggta atacgtatgg tgcaagcgtt atccggattt 180

actgggtgta aagggagcgc aggcggtgcg gcaagtctga tgtgaaagcc cggggctcaa 240

ccccggtact gcattggaaa ctgtcgtact agagtgtcgg aggggtaagc ggaattccta 300

gtgtagcggt gaaatgcgta gatattagga ggaacaccag tggcgaaggc ggcttactgg 360

acgataactg acgctgaggc tcgaaagcgt gggggagcaa ac 402

<210> 13
<211> 264
<212> DNA
<213> Bacterium uniformis JCM5828 3 EU136680

<400> 13

acggaaggca gcagtgagga atattggtca atggacgaga gtctgaacca gccaagtagc 60

gtgaaggatg actgccctat gggttgtaaa cttcttttat acgggaataa agtgaggcac 120

gtgtgccttt ttgtatgtac cgtatgaata aggatcggct aactccgtgc cagcagccgc 180

ggtaatacgg aggatccgag cgttatccgg atttattggg tttaaaggga gcgtaggcgg 240

acgcttaagt cagttgtgaa agtt 264

<210> 14
<211> 204
<212> DNA
<213> Bacteroides sp ALA Bac AM117579

<400> 14
acgggaggca gcagtgagga atattggtca atgggcgaga gcctgaacca gccaagtagc 60

gtgaaggatg actgccctat gggttgtaaa cttcttttat aaaggaataa agtcgggtat 120

gcatacccgt ttgcatgtac tttatgaata aggatcggct aactccgtgc cagcagccgc 180

ggtaatacgg aggatccgag cgtt 204

<210> 15
<211> 204
<212> DNA
<213> Bacteroides uniformis JCM 5828T AB50110

<400> 15
acgggaggca gcagtgagga atattggtca atggacgaga gtctgaacca gccaagtagc 60

gtgaaggatg actgccctat gggttgtaaa cttcttttat acgggaataa agtgaggcac 120

gtgtgccttt ttgtatgtac cgtatgaata aggatcggct aactccgtgc cagcagccgc 180

ggtaatacgg aggatccgag cgtt 204

<210> 16
<211> 324
<212> DNA
<213> Blautia obeum AY169419

<400> 16
tggggaatat tgcacaatgg gggaaaccct gatgcagcga cgccgcgtga aggaagaagt 60

atctcggtat gtaaacttct atcagcaggg aagatagtga cggtacctga ctaagaagcc 120

ccggctaact acgtgccagc agccgcggta atacgtaggg ggcaagcgtt atccggattt 180

actgggtgta aagggagcgt agacggtgtg gcaagtctga tgtgaaaggc atgggctcaa 240

cctgtggact gcattggaaa ctgtcatact tgagtgccgg aggggtaagc ggaattccta 300

gtgtagcggt gaaatgcgta gata 324

<210> 17
<211> 1436
<212> DNA

<213>  Parabacteroides distasonis

<400>  17
tatgaacgct agcgacaggc ttaacacatg caagtcgagg ggtagcgggg gtagcgatac      60

ccgccggcga ccggcgcacg ggtgagtaac gcgtatgcaa cttgcctatc agagggggat     120

aacccggcga aagtcggact aataccgcat gaagcagggg ccccgcatgg ggatatttgc     180

taaagattca tcgctgatag ataggcatgc gttccattag gcagttggcg gggtaacggc     240

ccaccaaacc gacgatggat aggggttctg agaggaaggt cccccacatt ggtactgaga     300

cacggaccaa actcctacgg gaggcagcag tgaggaatat tggtcaatgg ccgagaggct     360

gaaccagcca agtcgcgtga gggatgaagg ttctatggat cgtaaacctc ttttataagg     420

gaataaagtg cgggacgtgt cccgttttgt atgtacctta tgaataagga tcggctaact     480

ccgtgccagc agccgcggta atacggagga tccgagcgtt atccggattt attgggttta     540

aagggtgcgt aggcggcctt ttaagtcagc ggtgaaagtc tgtggctcaa ccatagaatt     600

gccgttgaaa ctggggggct tgagtatgtt tgaggcaggc ggaatgcgtg gtgtagcggt     660

gaaatgcata gatatcacgc agaaccccga ttgcgaaggc agcctgccaa gccattactg     720

acgctgatgc acgaaagcgt gggggatcaa acaggattag ataccctggt agtccacgca     780

gtaaacgatg atcactagct gtttgcgata cactgtaagc ggcacagcga aagcgttaag     840

tgatccacct ggggagtacg ccggcaacgg tgaaactcaa aggaattgac gggggcccgc     900

acaagcggag gaacatgtgg tttaattcga tgatacgcga ggaaccttac ccgggtttga     960

acgcattcgg accgaggtgg aaacaccttt tctagcaata gccgtttgcg aggtgctgca    1020

tggttgtcgt cagctcgtgc cgtgaggtgt cggcttaagt gccataacga gcgcaaccct    1080

tgccactagt tactaacagg taaagctgag gactctggtg ggactgccag cgtaagctgc    1140

gaggaaggcg gggatgacgt caaatcagca cggcccttac atccggggcg acacacgtgt    1200

tacaatggcg tggacaaagg gaggccacct ggcgacaggg agcgaatccc caaaccacgt    1260

ctcagttcgg atcggagtct gcaacccgac tccgtgaagc tggattcgct agtaatcgcg    1320

catcagccat ggcgcggtga atacgttccc gggccttgta cacaccgccc gtcaagccat    1380

gggagccggg ggtacctgaa gtccgtaacc gaaaggatcg gcctagggta aaactg        1436

<210>  18
<211>  1422
<212>  DNA
<213>  uncultured bacterium NA48 AY975552

<400>  18
ccttctaata gagtttgatc ctggctcagg atgaacgctg gcgcctttcc taacacatgc      60

aagtcgaacg gagctgcttt gatgaagttt tcggatggat ttaaaacagc ttagtggcgg     120

acgggtgagt aacgcgtggg taacctgcct cacactgggg gataacagtt agaaatagct     180

gctaataccg cataagcgca cagttccgca tggaacagtg tgaaaaactc cggtggtgtg 240

agatggaccc gcgtctgatt agccagttgg cggggtaacg gcccaccaaa gcgacgatca 300

gtagccggcc tgagagggtg aacggccaca ttgggactga gacacggccc aaactcctac 360

gggaggcagc agtggggaat attgcacaat gggggaaacc ctgatgcagc gacgccgcgt 420

gagtgaagaa gtatttcggt atgtaaagct ctatcagcag ggaagaaagt gacggtacct 480

gaataagaag ccccggctaa ctacgtgcca gcagccgcgg taatacgtag ggggcaagcg 540

ttatccggat ttactgggtg taaagggagc gtagacggca aggcaagtct gaagtgaaag 600

cccggtgctt aacgccggga ctgctttgga aactgtttgg ctggagtgcc ggagaggtaa 660

gcggaattcc tagtgtagcg gtgaaatgcg tagatattag gaagaacacc agtggcgaag 720

gcggcttact ggacggtaac tgacgttgag gctcgaaagc gtggggagca aacaggatta 780

gataccctgg tagtccacgc cgtaaacgat gattgctagg tgtaggtggg tatggaccca 840

tcggtgccgc agctaacgca ataagcaatc cacctggggg agtacgttcg caagaatgaa 900

actcaaagga attgacgggg acccgcacaa gcggtggagc atgtggttta attcgaagca 960

acgcgaagaa ccttaccagg tcttgacatc ccgatgaaaa acccggtaac ggggttccct 1020

ctttcggagc atccggagac aaggtggggg catgggtttg ccgccagctc ctggtcctgg 1080

aaaattttgg ggttaaatcc ccccaaccaa ccgcacaccc tttattcttt tgaaccccac 1140

aaggtaaagg cttgggcact cctaagagga actggcccgg ggataacccg gaggaaggtg 1200

gggatgacgt caaatcatca tgccccttat gatctgggct acacacgtgc tacaatggcg 1260

taacaaaggg aagcgagcct gcgagggtga gcaaatccca aaaataacgt cccagttcgg 1320

actgtagtct gcaacccgac tacacgaagc tggaatcgct agtaatcgcg aatcagaatg 1380

tcgcggtgaa tacgttcccg ggtcttgcac acaccgcccg tc 1422

<210> 19
<211> 1491
<212> DNA
<213> butyrate producing bacterium SS2 1 AY305319

<400> 19
agagtttgat cctggctcag gatgaacgct ggcggcgtgc ttaacacatg caagtcgaac 60

gaaacacctt atttgatttt cttcggaact gaagatttgg tgattgagtg gcggacgggt 120

gagtaacgcg tgggtaacct accctgtaca gggggataac agtcagaaat gactgctaat 180

accgcataag accacagcac cgcatggtgc aggggtaaaa actccggtgg tacaggatgg 240

acccgcgtct gattagctgg ttggtgaggt aacggctcac caaggcgacg atcagtagcc 300

ggcttgagag agtgaacggc cacattggga ctgagacacg gcccaaactc ctacgggagg 360

cagcagtggg gaatattgca caatggggga aaccctgatg cagcgacgcc gcgtgagtga 420

agaagtatct cggtatgtaa agctctatca gcagggaaga aaatgacggt acctgactaa 480

gaagccccgg ctaactacgt gccagcagcc gcggtaatac gtagggggca agcgttatcc 540

ggaattactg ggtgtaaagg gtgcgtaggt ggtatggcaa gtcagaagtg aaaacccagg 600

gcttaactct gggactgctt ttgaaactgt cagactggag tgcaggagag gtaagcggaa 660

ttcctagtgt agcggtgaaa tgcgtagata ttaggaggaa catcagtggc gaaggcggct 720

tactggactg aaactgacac tgaggcacga aagcgtgggg gagcaaacag gattagatac 780

cctggtagtc cacgccgtaa acgatgaata ctaggtgtcg gggccgtaga ggctccggtg 840

ccgcagccaa cgcagtaagt attccacctg gggagtacgc tcgcaagaat gaaactcaaa 900

ggaattgacg gggacccgca caagcggtgg agcatgtggt ttaattcgaa gcaacgcgaa 960

gaaccttacc tggtcttgac atccttctga ccggtcctta accggacctt tccttcggga 1020

caggagtgac aggtggtgca tggttgtcgt cagctcgtgt cgtgagatgt tgggttaagt 1080

cccgcaacga gcgcaacccc tatctttagt agccagcatt tcaggtgggc actctagaga 1140

gactgccagg gataacctgg aggaaggtgg ggacgacgtc aaatcatcat gccccttacg 1200

accagggcta cacacgtgct acaatggcgt aaacagaggg aagcagcctc gtgagagtga 1260

gcaaatccca aaaataacgt ctcagttcgg attgtagtct gcaactcgac tacatgaagc 1320

tggaatcgct agtaaccgcg aatcagaatg tcgcggtgaa tacgttcccg ggtcttgtac 1380

acaccgcccg tcacaccatg ggagtcagta acgcccgaag tcagtgaccc aaccgtaagg 1440

agggagctgc cgaaggcggg accgataact ggggtgaagt cgtaacaagg t 1491

<210> 20
<211> 1488
<212> DNA
<213> uncultured bacterium C3 13 GQ896957

<400> 20
ggttcgattc tggctcagga cgaacgctgg cggcgtgctt aacacatgca agtcgaacga 60

gaatcactgg aaggagtttt cggacaacgg aaggtgagga cagtggcgga cgggtgagta 120

acgcgtgagg aacctgcctt tcagaggggg acaacagttg gaaacgactg ctaataccgc 180

atgacacata gagatcgcat ggtttttatg tcaaagattt atcgctgaaa gatggcctcg 240

cgtctgatta gctagttggt gaggtaacgg cccaccaagg cgacgatcag tagccggact 300

gagaggttga ccggccacat tgggactgag atacggccca gactcctacg ggaggcagca 360

gtggggaata ttgggcaatg ggcgcaagcc tgacccagca acgccgcgtg aaggaagaag 420

gctttcgggt tgtaaacttc ttttgtcggg gacgagtaga agacggtacc tggcgaataa 480

gccacggcta actacgtgcc agcagccgcg gtaatacgta ggtggcaagc gttgtccgga 540

tttactgggt gtaaagggcg tgtagccggg aaggcaagtc agatgtgaaa tccacgggct 600

taactcgtga actgcatttg aaactacttt tcttgagtat cggagaggca atcggaattc 660

ctagtgtagc ggtgaaatgc gtagatatta ggaggaacac cagtggcgaa ggcggattgc 720

tggacgacaa ctgacggtga ggcgcgaaag cgtggggagc aaacaggatt agataccctg 780

gtagtccacg ctgtaaacga tgaatactag gtgtgcgggg actgaccccc tgcgtgccgc 840

agttaacaca ataagtattc cacctgggga gtacgatcgc aaggttgaaa ctcaaaggaa 900

ttgacggggg cccgcacaag cggtggatta tgtggtttaa ttcgacgcaa cgcgaagaac 960

cttaccaggg cttgacatcc tactaacgaa gtagagatac atcaggtgcc cttcggggaa 1020

agtagagaca ggtggtgcat ggttgtcgtc agctcgtgtc gtgagatgtt gggttaagtc 1080

ccgcaacgag cgcaacccct attgttagtt gctacgcaag agcactctag cgagactgcc 1140

gttgacaaaa cggaggaagg tggggacgac gtcaaatcat catgcccctt atgtcctggg 1200

ctacacacgt aatacaatgg cggtcaacag agggatgcaa aaccgtaagg tggagcgaac 1260

ccctaaaagc cgtctcagtt cagattgtgg gctgcaaccc gcccacatga agtcggaatc 1320

gctagtaatc gcggatcagc atgccgcggt gaatacgttc ccgggccttg tacacaccgc 1380

ccgtcacacc atgagagtcg ggaacacccg aagtccgtag cctaaccgta aggagggcgc 1440

ggccgaaggt gggttcgata attggggtga agtcgtaaca aggtaccc 1488

<210> 21
<211> 1372
<212> DNA
<213> Faecalibacterium prausnitzii

<400> 21
caagtcgaac gagagatgag gagcttgctc ttcagatcga gtggcgaacg ggtgagtaac 60

gcgtgaggaa cctgcctcaa agagggggac aacagttgga aacgactgct aataccgcat 120

aagcccacgg ctcggcatcg agcagaggga aaaggagtga tccgctttga gatggcctcg 180

cgtccgatta gctggttggt gaggtaacgg cccaccaagg cgacgatcgg tagccggact 240

gagaggttga acggccacat tgggactgag acacggccca gactcctacg ggaggcagca 300

gtggggaata ttgcacaatg ggggaaaccc tgatgcagcg acgccgcgtg gaggaagaag 360

gtcttcggat tgtaaactcc tgttgttgag gaagataatg acggtactca acaaggaagt 420

gacggctaac tacgtgccag cagccgcggt aaaacgtagg tcacaagcgt tgtccggaat 480

tactgggtgt aaagggagcg caggcgggag aacaagttgg aagtgaaatc catgggctca 540

acccatgaac tgctttcaaa actgtttttc ttgagtagtg cagaggtagg cggaattccc 600

ggtgtagcgg tggaatgcgt agatatcggg aggaacacca gtggcgaagg cggcctactg 660

ggcaccaact gacgctgagg ctcgaaagtg tgggtagcaa acaggattag ataccctggt 720

agtccacacc gtaaacgatg attactaggt gttggaggat tgaccccttc agtgccgcag 780

ttaacacaat aagtaatcca cctggggagt acgaccgcaa ggttgaaact caaaggaatt 840

gacgggggcc cgcacaagca gtggagtatg tggtttaatt cgacgcaacg cgaagaacct 900

taccaagtct tgacatccct tgacgaacat agaaatattt tttctcttcg gagcaaggag 960

acaggtggtg catggttgtc gtcagctcgt gtcgtgagat gttgggttaa gtcccgcaac 1020

gagcgcaacc cttatggtca gttactacgc aagaggactc tggccagact gccgttgaca 1080

aaacggagga aggtggggat gacgtcaaat catcatgccc tttatgactt gggctacaca 1140

cgtactacaa tggcgttaaa caaagagaag caagaccgcg aggtggagca aaactcagaa 1200

acaacgtccc agttcggact gcaggctgca actcgcctgc acgaagtcgg aattgctagt 1260

aatcgtggat cagcatgcca cggtgaatac gttcccgggc cttgtacaca ccgcccgtca 1320

caccatgaga gccgggggga cccgaagtcg gtagtctaac cgcaaggagg ac 1372

<210> 22
<211> 1426
<212> DNA
<213> Gemmiger formicilis

<400> 22
catgcagtcg acggagctag aggagcttgc ttttcttggc ttagtggcga acgggtgagt 60

aacgcgtgag taacctgccc tggagtgggg gacaacagtt ggaaacgact gctaataccg 120

cataagccca cgatccggca tcggatcgag ggaaaaggat tttttcgctt caggatggac 180

tcgcgtccaa ttagctagtt ggtgaggtaa cggcccacca aggcgacgat tggtagccgg 240

actgagaggt tgaacggcca cattgggact gagacacggc ccagactcct acgggaggca 300

gcagtggggg atattgcaca atgggggaaa ccctgatgca gcgacgccgc gtggaggaag 360

aaggttttcg gattgtaaac tcctgtcgtt agggacgata atgacggtac ctaacaagaa 420

agcaccggct aactacgtgc cagcagccgc ggtaaaacgt agggtgcaag cgttgtccgg 480

aattactggg tgtaaaggga gcgcaggcgg accggcaagt tggaagtgaa aactatgggc 540

tcaacccata aattgctttc aaaactgctg gccttgagta gtgcagaggt aggtggaatt 600

cccggtgtag cggtggaatg cgtagatatc gggaggaaca ccagtggcga aggcgaccta 660

ctgggcacca actgacgctg aggctcgaaa gcatgggtag caaacaggat tagataccct 720

ggtagtccat gccgtaaacg atgattacta ggtgttggag gattgacccc ttcagtgccg 780

cagttaacac aataagtaat ccacctgggg agtacgaccg caaggttgaa actcaaagga 840

attgacgggg gcccgcacaa gcagtggagt atgtggttta attcgaagca acgcgaagaa 900

ccttaccagg tcttgacatc cgatgcatag cgcagagatg catgaagtcc ttcgggacat 960

cgagacaggt ggtgcatggt tgtcgtcagc tcgtgtcgtg agatgttggg ttaagtcccg 1020

caacgagcgc aacccttatt gccagttact acgcaagagg actctggcga gactgccgtt 1080

gacaaaacgg aggaaggtgg ggatgacgtc aaatcatcat gccctttatg acctgggcta 1140

cacacgtact acaatggcgt ttaacaaaga gaagcaagac cgcgaggtgg agcaaaactc 1200

aaaaacaacg tctcagttca gattgcaggc tgcaactcgc ctgcatgaag tcggaattgc 1260

tagtaatcgc ggatcagcat gccgcggtga atacgttccc gggccttgta cacaccgccc 1320

gtcacaccat gagagccggg gggacccgaa gtcggtagtc taaccgcaag gaggacgccg 1380

ccgaagtaaa actggtgatt ggggtgaagt cgtacagggg gtaacc 1426

<210> 23
<211> 1471
<212> DNA
<213> uncultured bacterium RL246 aai74e12 DQ793623

<400> 23
ggttcgattc tggctcagga cgaacgctgg cggcgcgcct aacacatgca agtcgaacga 60

gcgagagaga gcttgctttc tcgagcgagt ggcgaacggg tgagtaacgc gtgaggaacc 120

tgcctcaaag agggggacaa cagttggaaa cgactgctaa taccgcataa gcccacggct 180

cggcatcgag cagggggaaa aggagcaatc cgctttgaga tggcctcgcg tccgattagc 240

tagttggtga ggtaacggcc caccaaggca acgatcggta gccagactga gaggttgaac 300

ggccacattg ggactgagac acggcccaga ctcctacggg aggcagcagt ggggaatatt 360

gcacaatggg ggaaaccctg atgcagcgac gccgcgtgga ggaagaaggt cttcggattg 420

taaactcctg ttgttgggga agataatgac ggtacccaac aaggaagtga cggctaacta 480

cgtgccagca gccgcggtaa aacgtaggtc acaagcgttg tccggaatta ctgggtgtaa 540

agggagcgca ggcgggaaga caagttggaa gtgaaatcta tgggctcaac ccataaactg 600

ctttcaaaac tgtttttctt gagtagtgca gaggtaggcg gaattcccgg tgtagcggtg 660

gaatgcgtag atatcgggag gaacaccagt ggcgaaggcg gcctactggg caccaactga 720

cgctgaggct cgaaagtgtg gggtagcaaa caggattaga taccctggta gtccacaccg 780

taaacgatga ttactaggtg ttggaggatt gaccccttca gtgccgcagt taacacaata 840

agtaatccac ctggggagta cgaccgcaag gttgaaactc aaaggaattg acgggggccc 900

gcacaagcag tggagtatgt ggtttaattc gacgcaacgc gaagaacctt accaagtctt 960

gacatccctt gacaggcata gaaatatgtt ttctcttcgg agcaaggaga caggtggtgc 1020

atggttgtcg tcagctcgtg tcgtgagatg ttgggttaag tcccgcaacg agcgcaaccc 1080

ttatggtcag ttactacgca agaggactct ggccagactg ccgttgacaa aacggaggaa 1140

ggtggggatg acgtcaaatc atcatgccct ttatgacttg ggctacacac gtactacaat 1200

ggcgttaaac aaagagaagc aagaccgcga ggtggagcaa aactcagaaa caacgtccca 1260

gttcggactg caggctgcaa ctcgcctgca cgaagtcgga attgctagta atcgtggatc 1320

agcatgccac ggtgaatacg ttcccgggcc ttgtacacac cgcccgtcac accatgagag 1380

ccggggggac ccgaagtcgg tagtctaacc gcaaggagga cgccgccgaa ggtaaaactg 1440

gtgattgggg tgaagtcgta acaaggtacc c 1471

<210> 24
<211> 1383
<212> DNA
<213> Bacteroides ovatus

<400> 24
cagtcgaggg gcagcatttt agtttgcttg caactgaaga tggcgaccgg cgcacgggtg 60

agtaacacgt atccaacctg ccgataactc cggaatagcc tttcgaaaga aagattaata 120

ccggatagca tacgaatatc gcatgatatt tttattaaag aatttcggtt atcgatgggg 180

atgcgttcca ttagtttgtt ggcggggtaa cggcccacca agactacgat ggataggggt 240

tctgagagga aggtccccca cattggaact gagacacggt ccaaactcct acgggaggca 300

gcagtgagga atattggtca atgggcgaga gcctgaacca gccaagtagc gtgaaggatg 360

aaggctctat gggtcgtaaa cttcttttat atgggaataa agttttccac gtgtggaatt 420

ttgtatgtac catatgaata aggatcggct aactccgtgc cagcagccgc ggtaatacgg 480

aggatccgag cgttatccgg atttattggg tttaaaggga gcgtaggtgg attgttaagt 540

cagttgtgaa agtttgcggc tcaaccgtaa aattgcagtt gaaactggca gtcttgagta 600

cagtagaggt gggcggaatt cgtggtgtag cggtgaaatg cttagatatc acgaagaact 660

ccgattgcga aggcagctca ctagactgtc actgacactg atgctcgaaa gtgtgggtat 720

caaacaggat tagataccct ggtagtccac acagtaaacg atgaatactc gctgtttgcg 780

atatacagta agcggccaag cgaaagcatt aagtattcca cctggggagt acgccggcaa 840

cggtgaaact caaaggaatt gacgggggcc cgcacaagcg gaggaacatg tggtttaatt 900

cgatgatacg cgaggaacct tacccgggct taaattgcaa cagaatatat tggaaacagt 960

atagccgtaa ggctgttgtg aaggtgctgc atggttgtcg tcagctcgtg ccgtgaggtg 1020

tcggcttaag tgccataacg agcgcaaccc ttatctttag ttactaacag gtcatgctga 1080

ggactctaga gagactgccg tcgtaagatg tgaggaaggt ggggatgacg tcaaatcagc 1140

acggccctta cgtccggggc tacacacgtg ttacaatggg gggtacagaa ggcggctacc 1200

tggtgacagg atgctaatcc caaaaacctc tctcagttcg gatcgaagtc tgcaacccga 1260

cttcgtgaag ctggattcgc tagtaatcgc gcatcagcca tggcgcggtg aatacgttcc 1320

cgggccttgt acacaccgcc cgtcaagcca tgaaagccgg gggtacctga agtacgtaac 1380

cgc 1383

<210>  25
<211>  1372
<212>  DNA
<213>  Faecalibacterium prausnitzii

<400>  25
caagtcgaac gagagatgag gagcttgctc ttcagatcga gtggcgaacg ggtgagtaac      60

gcgtgaggaa cctgcctcaa agagggggac aacagttgga aacgactgct aataccgcat     120

aagcccacgg ctcggcatcg agcagaggga aaaggagtga tccgctttga gatggcctcg     180

cgtccgatta gctggttggt gaggtaacgg cccaccaagg cgacgatcgg tagccggact     240

gagaggttga acggccacat tgggactgag acacggccca gactcctacg ggaggcagca     300

gtggggaata ttgcacaatg ggggaaaccc tgatgcagcg acgccgcgtg gaggaagaag     360

gtcttcggat tgtaaactcc tgttgttgag gaagataatg acggtactca acaaggaagt     420

gacggctaac tacgtgccag cagccgcggt aaaacgtagg tcacaagcgt tgtccggaat     480

tactgggtgt aaagggagcg caggcgggag aacaagttgg aagtgaaatc catgggctca     540

acccatgaac tgctttcaaa actgtttttc ttgagtagtg cagaggtagg cggaattccc     600

ggtgtagcgg tggaatgcgt agatatcggg aggaacacca gtggcgaagg cggcctactg     660

ggcaccaact gacgctgagg ctcgaaagtg tgggtagcaa acaggattag ataccctggt     720

agtccacacc gtaaacgatg attactaggt gttggaggat tgaccccttc agtgccgcag     780

ttaacacaat aagtaatcca cctggggagt acgaccgcaa ggttgaaact caaaggaatt     840

gacgggggcc cgcacaagca gtggagtatg tggtttaatt cgacgcaacg cgaagaacct     900

taccaagtct tgacatccct tgacgaacat agaaatattt tttctcttcg gagcaaggag     960

acaggtggtg catggttgtc gtcagctcgt gtcgtgagat gttgggttaa gtcccgcaac    1020

gagcgcaacc cttatggtca gttactacgc aagaggactc tggccagact gccgttgaca    1080

aaacggagga aggtggggat gacgtcaaat catcatgccc tttatgactt gggctacaca    1140

cgtactacaa tggcgttaaa caaagagaag caagaccgcg aggtggagca aaactcagaa    1200

acaacgtccc agttcggact gcaggctgca actcgcctgc acgaagtcgg aattgctagt    1260

aatcgtggat cagcatgcca cggtgaatac gttcccgggc cttgtacaca ccgcccgtca    1320

caccatgaga gccgggggga cccgaagtcg gtagtctaac cgcaaggagg ac            1372

<210>  26
<211>  1495
<212>  DNA
<213>  Eisenbergiella massiliensis

<400>  26
agagtttgat cctggctcag gatgaacgct ggcggcgtgc ctaacacatg caagtcgaac      60

gragttatgc agaggaagtt ttcggatgga atcggcgtaa cttagtggcg gacgggtgag     120

taacgcgtgg gaaacctgcc ctgtaccggg ggataacact tagaaatagg tgctaatacc 180

gcataagcgc acagcytcac atgargcagt gtgaaaaact ccggtggtac aggatggtcc 240

cgcgtctgat tagccagttg gcagggtrac ggcctaccaa agcgacgatc agtagccggc 300

ctgagagggt gaacggccac attgggactg agacacggcc caaactccta cgggaggcag 360

cagtggggaa tattgcacaa tgggggaaac cctgatgcag cgacgccgcg tgagtgaaga 420

agtatttcgg tatgtaaagc tctatcagca gggaagaaaa tgacggtacc tgactaagaa 480

gccccggcta actacgtgcc agcagccgcg gtaatacgta gggggcaagc gttatccgga 540

tttactgggt gtaaagggag cgtagacggc atgacaagcc agatgtgaaa acccagggct 600

caaccctggg actgcatttg gaactgccag gctggagtgc aggagaggta agcggaattc 660

ctagtgtagc ggtgaaatgc gtagatatta ggaggaacac cagtggcgaa ggcggcttac 720

tggactgtaa ctgacgttga ggctcgaaag cgtggggagc aaacaggatt agataccctg 780

gtagtccacg cggtaaacga tgattgctag gtgtaggtgg gtatggaccc atcggtgccg 840

cagctaacgc aataagcaat ccacctgggg agtacgttcg caagaatgaa actcaaagga 900

attgacgggg acccgcacaa gcggtggagc atgtggttta attcgaagca acgcgaagaa 960

ccttaccaag tcttgacatc ccaatgacgt gtccgtaayg gggcattctc ttcggagcat 1020

tggagacagg tggtgcatgg ttgtcgtcag ctcgtgtcgt gagatgttgg gttaagtccc 1080

gcaacgagcg caacccttat ccttagtagc cagcaggtaa agctgggcac tctagggaga 1140

ctgccgggga taacccggag gaaggcgggg atgacgtcaa atcatcatgc cccttatgat 1200

ttgggctaca cacgtgctac aatggcgtaa acaaagggaa gcgagacagt gatgttgagc 1260

aaatcccaga aataacgtct cagttcggat tgtagtctgc aactcgacta catgaagctg 1320

gaatcgctag taatcgcgaa tcagcatgtc gcggtgaata cgttcccggg tcttgtacac 1380

accgcccgtc acaccatggg agttggaaat gcccgaagcc tgtgacctaa ccgcaaggga 1440

ggagcagtcg aaggcaggtc taataactgg ggtgaagtcg taacaaggta gccgt 1495

<210> 27
<211> 1451
<212> DNA
<213> uncultured bacterium REC1M 21 AY343160

<400> 27
gacgaacgct ggcggcgtgc ttaacacatg caagtcgaac gagaatctac ggatcgagga 60

ttcgtccaag tgaagtagag gacagtggcg gacgggtgag taacgcgtga ggaacctgcc 120

tttcagaggg ggacaacagt tggaaacgac tgctaatacc gcatgacaca ttggagtcgc 180

atggctctga tgtcaaagat ttatcgctga aagatggcct cgcgtctgat tagctagttg 240

gtgaggtaac ggcccaccaa ggcgacgatc agtagccgga ctgagaggtt gaccggccac 300

attgggactg agatacggcc cagactccta cgggaggcag cagtggggaa tattgggcaa 360

tgggcgcaag cctgacccag caacgccgcg tgaaggaaga aggctttcgg gttgtaaact 420

tcttttgtca gggacgagta gaagacggta cctgacgaat aagccacggc taactacgtg 480

ccagcagccg cggtaatacg taggtggcaa gcgttgtccg gatttactgg gtgtaaaggg 540

cgtgtagccg ggaaggcaag tcagatgtga aatccacggg ctcaactcgt gaactgcatt 600

tgaaactgtt tttcttgagt atcggagagg caatcggaat tcctagtgta gcggtgaaat 660

gcgtagatat taggaggaac accagtggcg aaggcggatt gctggacgac aactgacggt 720

gaggcgcgaa agcgtgggga gcaaacagga ttagataccc tggtagtcca cgctgtaaac 780

gatgaatact aggtgtgcgg ggactgaccc cctgcgtgcc gcagttaaca caataagtat 840

tccacctggg gagtacgatc gcaaggttga aactcaaagg aattgacggg gacccgcaca 900

agcggtggat tatgtggttt aattcgaagc aacgcgaaga accttaccag ggcttgacat 960

cctactaacg agatagagat atgttaggtg cccttcgggg aaagtagaga caggtggtgc 1020

atggttgtcg tcagctcgtg tcgtgagatg ttgggttaag tcccgcaacg agcgcaaccc 1080

ctattgttag ttgctacgca agagcactct agcgagactg ccgttgacaa aacggaggaa 1140

ggtggggacg acgtcaaatc atcatgcccc ttatgtcctg ggctacacac gtaatacaat 1200

ggcggtcaac agagggatgc gataccgcaa ggtggagcga acccctaaaa gccgtcccag 1260

ttcagattgt gggctgcaac ccgcccacat gaagtcggaa tcgctagtaa tcgcagatca 1320

gcatgctgcg gtgaatacgt tcccgggcct tgtacacacc gcccgtcaca ccatgagagt 1380

cgggaacacc cgaagcctgt agcctaacca ttcggagggc gcagtcgaag gtgggttcga 1440

taattggggt g 1451

<210> 28
<211> 1447
<212> DNA
<213> butyrate producing bacterium L2 21 AJ270477

<400> 28
gctcaggatg aacgctggcg gcgtgcttaa cacatgcaag tcgaacgaag cactttattt 60

gatttccttc gggactgatt attttgtgac tgagtggcgg acgggtgagt aacgcgtggg 120

taacctgcct tgtacagggg gataacagtt ggaaacggct gctaataccg cataagcgca 180

cagcatcgca tgatgctgtg tgaaaaactc cggtggtata agatggaccc gcgttggatt 240

agctagttgg tgaggtaacg gcccaccaag gcgacgatcc atagccgacc tgagagggtg 300

accggccaca ttgggactga gacacggccc aaactcctac gggaggcagc agtggggaat 360

attgcacaat gggcgaaagc ctgatgcagc gacgccgcgt gagcgaagaa gtatttcggt 420

atgtaaagct ctatcagcag ggaagataat gacggtacct gactaagaag caccggctaa 480

atacgtgcca gcagccgcgg taatacgtat ggtgcaagcg ttatccggat ttactgggtg 540

taaagggagc gcaggcggtg cggcaagtct gatgtgaaag cccggggctc aaccccggta 600

ctgcattgga aactgtcgta ctagagtgtc ggaggggtaa gcggaattcc tagtgtagcg 660

gtgaaatgcg tagatattag gaggaacacc agtggcgaag gcggcttact ggacgataac 720

tgacgctgag gctcgaaagc gtgggggagc aaacaggatt agataccctg gtagtccacg 780

ccgtaaacga tgaatactag gtgttgggaa gcattgcttc tcggtgccgt cgcaaacgca 840

gtaagtattc cacctgggga gtacgttcgc aagaatgaaa ctcaaaggaa ttgacgggga 900

cccgcacaag cggtggagca tgtggtttaa ttcgaagcaa cgcgaagaac cttaccaagt 960

cttgacatcc ttctgaccgg tacttaatcg taccttctct tcggagcagg agtgacaggt 1020

ggtgcatggt tgtcgtcagc tcgtgtcgtg agatgttggg ttaagtcccg caacgagcgc 1080

aacccttatc tttagtagcc agcggtcagg ccgggcactc tagagagact gccagggata 1140

acttggagga aggcggggat gacgtcaaat catcatgccc cttatgactt gggctacaca 1200

cgtgctacaa tggcgtaaac aaagggaagc aaagctgtga agccgagcaa atctcaaaaa 1260

taacgtctca gttcggactg tagtctgcaa cccgactaca cgaagctgga atcgctagta 1320

atcgcagatc agaatgctgc ggtgaatacg ttcccgggtc ttgtacacac cgcccgtcac 1380

accatgggag ttgggaatgc ccgaagccag tgacctaacc gaaaggaagg agctgtcgaa 1440

ggcaggc 1447

<210> 29
<211> 1477
<212> DNA
<213> Bacterium uniformis JCM5828 3 EU136680

<400> 29
ctggctcagg atgaacgcta gctacaggct taacacatgc aagtcgaggg gcagcatgaa 60

cttagcttgc taagtttgat ggcgaccggc gcacgggtga gtaacacgta tccaacctgc 120

cgatgactcg gggatagcct ttcgaaagaa agattaatac ccgatggcat agttcttccg 180

catggtagaa ctattaaaga atttcggtca tcgatgggga tgcgttccat taggttgttg 240

gcggggtaac ggcccaccaa gccttcgatg gataggggtt ctgagaggaa ggtcccccac 300

attggaactg agacacggtc caaactccta cgggaggcag cagtgaggaa tattggtcaa 360

tggacgagag tctgaaccag ccaagtagcg tgaaggatga ctgccctatg ggttgtaaac 420

ttcttttata cgggaataaa gtgaggcacg tgtgcctttt tgtatgtacc gtatgaataa 480

ggatcggcta actccgtgcc agcagccgcg gtaatacgga ggatccgagc gttatccgga 540

tttattgggt ttaaagggag cgtaggcgga cgcttaagtc agttgtgaaa gtttgcggct 600

caaccgtaaa attgcagttg atactgggtg tcttgagtac agtagaggca ggcggaattc 660

gtggtgtagc ggtgaaatgc ttagatatca cgaagaactc cgattgcgaa ggcagcttgc 720

tggactgtaa ctgacgctga tgctcgaaag tgtgggtatc aaacaggatt agataccctg 780

gtagtccaca cagtaaacga tgaatactcg ctgtttgcga tatacagtaa gcggccaagc 840

gaaagcgtta agtattccac ctggggagta cgccggcaac ggtgaaactc aaaggaattg 900

acgggggccc gcacaagcgg aggaacatgt ggtttaattc gatgatacgc gaggaacctt 960

acccgggctt gaattgcaac tgaatgatgt ggagacatgt cagccgcaag gcagttgtga 1020

aggtgctgca tggttgtcgt cagctcgtgc cgtgaggtgt cggcttaagt gccataacga 1080

gcgcaaccct tatcgatagt taccatcagg ttatgctggg gactctgtcg agactgccgt 1140

cgtaagatgt gaggaaggtg gggatgacgt caaatcagca cggcccttac gtccggggct 1200

acacacgtgt tacaatgggg ggtacagaag gcagctacac ggcgacgtga tgctaatccc 1260

taaagcctct ctcagttcgg attggagtct gcaacccgac tccatgaagc tggattcgct 1320

agtaatcgcg catcagccac ggcgcggtga atacgttccc gggccttgta cacaccgccc 1380

gtcaagccat gaaagccggg ggtacctgaa gtgcgtaacc gcaaggagcg ccctagggta 1440

aaactggtga ttggggctaa gtcgtaacaa ggtaacc 1477

<210> 30
<211> 1416
<212> DNA
<213> Bacteroides sp ALA Bac AM117579

<400> 30
ctggctcagg atgaacgcta gctacaggct taacacatgc aagtcgaggg gcagcatggt 60

cttagcttgc taaggccgat ggcgaccggc gcacgggtga gtaacacgta tccaacctgc 120

cgtctactct tggacagcct tctgaaagga agattaatac aagatggcat catgagtccg 180

catgttcaca tgattaaagg tattccggta gacgatgggg atgcgttcca ttagatagta 240

ggcggggtaa cggcccacct agtcttcgat ggataggggt tctgagagga aggtccccca 300

cattggaact gagacacggt ccaaactcct acgggaggca gcagtgagga atattggtca 360

atgggcgaga gcctgaacca gccaagtagc gtgaaggatg actgccctat gggttgtaaa 420

cttcttttat aaaggaataa agtcgggtat gcatacccgt ttgcatgtac tttatgaata 480

aggatcggct aactccgtgc cagcagccgc ggtaatacgg aggatccgag cgttatccgg 540

atttattggg tttaaaggga gcgtagatgg atgtttaagt cagttgtgaa agtttgcggc 600

tcaaccgtaa aattgcagtt gatactggat atcttgagtg cagttgaggc aggcggaatt 660

cgtggtgtag cggtgaaatg cttagatatc acgaagaact ccgattgcga aggcagcctg 720

ctaagctgca actgacattg aggctcgaaa gtgtgggtat caaacaggat tagataccct 780

ggtagtccac acggtaaacg atgaatactc gctgtttgcg atatactgca agcggccaag 840

cgaaagcgtt aagtattcca cctggggagt acgccggcaa cggtgaaact caaaggaatt 900

gacgggggcc cgcacaagcg gaggaacatg tggtttaatt cgatgatacg cgaggaacct 960

tacccgggct taaattgcag atgaattacg gtgaaagccg taagccgcaa ggcatctgtg 1020

aaggtgctgc atggttgtcg tcagctcgtg ccgtgaggtg tcggcttaag tgccataacg 1080

agcgcaaccc ttgttgtcag ttactaacag gttccgctga ggactctgac aagactgcca 1140

tcgtaagatg tgaggaaggt ggggatgacg tcaaatcagc acggccctta cgtccggggc 1200

tacacacgtg ttacaatggg gggtacagag ggccgctacc acgcgagtgg atgccaatcc 1260

ccaaaacctc tctcagttcg gactggagtc tgcaacccga ctccacgaag ctggattcgc 1320

tagtaatcgc gcatcagcca cggcgcggtg aatacgttcc cgggccttgt acacaccgcc 1380

cgtcaagcca tgggagccgg gggtacctga agtgcg 1416

<210> 31
<211> 1425
<212> DNA
<213> Bacteroides uniformis JCM 5828T AB50110

<400> 31
gtttgatcat ggctcaggat gaacgctagt acaggcttaa cacatgcaag tcgaggggca 60

gcatgaactt agcttgctaa gtttgatggc gaccggcgca cgggtgagta acacgtatcc 120

aacctgccga tgactcgggg atagcctttc gaaagaaaga ttaatacccg atggcatagt 180

tcttccgcat ggtagaacta ttaaagaatt tcggtcatcg atggggatgc gttccattag 240

gttgttggcg gggtaacggc ccaccaagcc ttcgatggat aggggttctg agaggaaggt 300

cccccacatt ggaactgaga cacggtccaa actcctacgg gaggcagcag tgaggaatat 360

tggtcaatgg acgagagtct gaaccagcca agtagcgtga aggatgactg ccctatgggt 420

tgtaaacttc ttttatacgg gaataaagtg aggcacgtgt gcctttttgt atgtaccgta 480

tgaataagga tcggctaact ccgtgccagc agccgcggta atacggagga tccgagcgtt 540

atccggattt attgggttta aagggagcgt aggcggacgc ttaagtcagt tgtgaaagtt 600

tgcggctcaa ccgtaaaatt gcagttgata ctgggtgtct tgagtacagt agaggcaggc 660

ggaattcgtg gtgtagcggt gaaatgctta gatatcacga agaactccga ttgcgaaggc 720

agcttgctgg actgtaactg acgctgatgc tcgaaagtgt gggtatcaaa caggattaga 780

taccctggta gtccacacag taaacgatga atactcgctg tttgcgatat acagtaagcg 840

gccaagcgaa agcgttaagt attccacctg gggagtacgc cggcaacggt gaaactcaaa 900

ggaattgacg ggggcccgca caagcggagg aacatgtggt ttaattcgat gatacgcgag 960

gaaccttacc cgggcttgaa ttgcaactga atgatgtgga gacatgtcag ccgcaaggca 1020

gttgtgaagg tgctgcatgg ttgtcgtcag ctcgtgccgt gaggtgtcgg cttaagtgcc 1080

ataacgagcg caacccttat cgatagttac catcaggtta tgctggggac tctgtcgaga 1140

ctgccgtcgt aagatgtgag gaaggtgggg atgacgtcaa atcagcacgg cccttacgtc 1200

cggggctaca cacgtgttac aatggggggt acagaaggca gctacacggc gacgtgatgc 1260

taatccctaa agcctctctc agttcggatt ggagtctgca acccgactcc atgaagctgg 1320

attcgctagt aatcgcgcat cagccacggc gcggtgaata cgttcccggg ccttgtacac 1380

accgcccgtc aagccatgaa agccgggggt acctgaagtg cgtaa 1425

<210> 32
<211> 1439
<212> DNA
<213> Blautia obeum AY169419

<400> 32
agagtttgat cctggctcag gatgaacgct ggcggcgtgc ttaacacatg caagtcgaac 60

gggaaatayt tyattgarac ttcggtsgat ttratytawt tctagtggcg gacgggtgag 120

taacgcgtgg gtaacctgcc ttatacaggg ggataacagt cagaaatggc tgctaatacc 180

gcataagcgc acagagctgc atggctcagt gtgaaaaact ccggtggtat aagatggacc 240

cgcgttggat tagctwgttg gtggggtaac ggcccaccaa ggcgacgatc catagccggc 300

ctgagagggt gaacggccac attgggactg agacacggcc cagactccta cgggaggcag 360

cagtggggaa tattgcacaa tgggggaaac cctgatgcag cgacgccgcg tgaaggaaga 420

agtatctcgg tatgtaaact tctatcagca gggaagatag tgacggtacc tgactaagaa 480

gccccggcta actacgtgcc agcagccgcg gtaatacgta gggggcaagc gttatccgga 540

tttactgggt gtaaagggag cgtagacggt gtggcaagtc tgatgtgaaa ggcatgggct 600

caacctgtgg actgcattgg aaactgtcat acttgagtgc cggaggggta agcggaattc 660

ctagtgtagc ggtgaaatgc gtagatatta ggaggaacac cagtggcgaa ggcggcttac 720

tggacggtaa ctgacgttga ggctcgaaag cgtggggagc aaacaggatt agataccctg 780

gtagtccacg ccgtaaacga tgaatactag gtgtcgggkr gcatrgcymt tcggtgccgt 840

cgcaaacgca gtaagtattc cacctgggga gtacgttcgc aagaatgaaa ctcaaaggaa 900

ttgacgggga cccgcacaag cggtggagca tgtggtttaa ttcgaagcaa cgcgaagaac 960

cttaccaart cttgacatcc syctgaccgr tcyttaaycg gaccttyyct tcggrrcagr 1020

sgwgacaggt ggtgcatggt tgtcgtcagc tcgtgtcgtg agatgttggg ttaagtcccg 1080

caacgagcgc aacccctatc ctcagtagcc agcatttaag gtgggcactc tggggagact 1140

gccagggata acctggagga aggcggggat gacgtcaaat catcatgccc cttatgattt 1200

gggctacaca cgtgctacaa tggcgtaaac aaagggaagc gagattgtga gatggagcaa 1260

atcccaaaaa taacgtccca gttcggactg tagtctgcaa cccgactaca cgaagctgga 1320

atcgctagta atcgcggatc agaatgccgc ggtgaatacg ttcccgggtc ttgtacacac 1380

cgcccgtcac accatgggag tcagtaacgc ccgaagtcag tgacctaact gcaaagaag 1439

## Description

The present invention relates to the field of anticancer treatment, more specifically to methods for *ex vivo* determining whether a patient with metastatic melanoma is likely to benefit from a treatment with an anti CTLA-4 molecule, preferably ipilimumab, by analysing the gut microbiota in a fecal sample from said patient.

Immunotherapy has become a major therapeutic strategy in oncology. The trail was blazed by an antibody directed against Cytotoxic T-lymphocyte-associated antigen 4 (CTLA-4), namely ipilimumab, which demonstrated a significant survival benefit in patients with metastatic melanoma (MM).^{1,2} It has previously been reported that 22% of MM patients treated with ipilimumab have a prolonged survival.³

CTLA-4 is a homolog of CD28; it down regulates T cell costimulation during antigen presentation, and thus constitutes a non-specific negative checkpoint of the immune response.⁴⁻⁶ Ipilimumab reactivates T cells that are primed in lymphoid organs, regardless of the antigen specificity, and therefore affects a much wider repertoire of T cells than those involved in the anti-cancer response.^{7,8} This probably explains why CTLA-4 blockade is associated with numerous and various immune-related adverse events (irAE). These irAE mainly affect the skin, the gut, the endocrine glands and the liver.^{9,10} Colitis affects 8 to 22% of patients treated with ipilimumab^{10,11} and requires discontinuation of ipilimumab and high dose steroids. Some patients with severe colitis may need anti-TNF therapy and sometimes colectomy.^{11,12} In summary, ipilimumab benefits a limited percentage of patients and can cause potentially severe irAE. In this context, reliable biomarkers of efficacy and/or toxicity capable of optimizing the risk/benefit ratio of this drug would be of paramount interest.

Colitis due to ipilimumab shares several features with inflammatory bowel disease (IBD).¹² In IBD, deregulated inflammation is associated with a dysbiotic composition of the intestinal microbiota, which is supposed to be the antigenic drive of the disease.¹³⁻¹⁵ The trillions of bacteria that constitute the human gut microbiota participate in the proper maturation of both mucosal and systemic immune system, and it has long been known that the composition of the vertebrates' microbiome was mandatory for the promotion of an appropriate immune response against pathogens and the maintenance of immune homeostasis.¹⁶ In addition, studies performed in mice have suggested that the microbiome composition was critical to promote an anti-tumor immune response to anti CTLA-4 and anti PD-1 monoclonal antibodies.^{17,18}

Inventors have conducted prospective study on patients with metastatic melanoma treated with ipilimumab. Fecal microbiota composition was assessed at baseline and before each ipilimumab infusion. Microbiota was studied using 16S rRNA gene sequencing; patients were clustered based on dominant microbiota pattern.

Based on this study, Inventors have now demonstrated that anticancer response as well as immune-related enterocolitis due to ipilimumab depend on microbiome composition.

Indeed results presented in the experimental part show that a distinct baseline gut microbiota composition was associated with both clinical response and colitis. As compared to patients whose baseline microbiota was driven by *Bacteroides* (cluster B, n=12), patients whose baseline microbiota was enriched with *Faecalibacterium* genus and other *Firmicutes* (cluster A, n=10) had longer progression-free survival (p=0.0039) and overall survival (p=0.051). Most of the baseline colitis-associated phylotypes were related to *Firmicutes* (eg, relatives of *Faecalibacterium prausnitzii* and *Gemmiger formicilis),* whereas no-colitis related phylotypes were assigned to *Bacteroidetes.* A low proportion of peripheral blood regulatory T cells was associated with cluster A, long-term clinical benefit and colitis. Ipilimumab led to a higher Inducible T-cell COStimulator induction on CD4+ T cells and to a higher increase in serum CD25 in patients who belonged to cluster A.

Baseline gut microbiota enriched with *Faecalibacterium* and other *Firmicutes* is associated with beneficial clinical response to ipilimumab and more frequent occurrence of ipilimumab-associated colitis.

The present invention thus relates to a method for predicting the response of an individual to an anticancer treatment with anti-CTL4 molecules comprising the steps of:
(i) determining gut microbial OTU (or phylotype or molecular species; i.e any group of 16S rRNA gene sequences sharing at least 97% of similarity between each other and hence grouped together in a taxonomic entity called an OTU : Operational Taxonomic Unit; and represented by a selected representative sequence) in a fecal sample of said individual;
(ii) determining a relative abundance of at least one OTU comprising a nucleotide fragment of sequence having at least 97%, preferably 98%, 99% or 100%, identity with a nucleic sequence selected in the group consisting of: denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7, denovo4054 of SEQ. ID. N°8, denovo3816 of SEQ. ID. N°9, denovo3657 of SEQ. ID. N°10, denovo5800 of SEQ. ID. N°11 and denovo5178 of SEQ. ID. N°12; preferably, determining a relative abundance of at least one OTU comprising a nucleotide fragment of sequence having at least 97%, preferably 98%, 99% or 100%, identity with a nucleic sequence selected in the group consisting of: denovo3066 of SEQ. ID. N°1, denovo991 of SEQ. ID. N°2, denovo3795 of SEQ. ID. N°5, denovo4787 of SEQ. ID. N°6, denovo4054 of SEQ. ID. N°8, denovo3816 of SEQ. ID. N°9, denovo3657 of SEQ. ID. N°10 and denovo5178 of SEQ. ID. N°12;
wherein individual having a gut microbiote enriched in at least one OTU selected in the group consisting of denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7 and denovo5178 of SEQ. ID. N°12 and/or depleted in at least one OTU selected in the group consisting of denovo4054 of SEQ. ID. N°8, denovo3816 of SEQ. ID. N°9, denovo3657 of SEQ. ID. N°10 and denovo5800 of SEQ. ID. N°11 is a good responder to said anticancer treatment.

The "relative abundance" of an OTU is defined as a percentage of the number of sequences grouped into this OTU to the total number of sequences in a fecal sample. OTU should group at least two 16S rRNA gene sequences (singletons, i.e. OTU containing only one sequence, are not considered).

The determination of gut microbial composition and of the relative abundance of a given OTU can be determined with classical and appropriate method known by the person skilled in the art. In a particular embodiment, said determination is performed on total DNA extracted from human fecal or mucosal or tissue sample by 16S rRNA gene sequencing, such as Sanger, 454 pyrosequencing, MiSeq or HiSeq technologies. Determination can also be performed by quantitative PCR technology with specific probes targeting either the specific OTU sequence or its affiliated bacterial isolate and any sequences having at least 97%, preferably 98%, 99% or 100%, identity with the nucleic sequence of said OTU.

Enrichment or depletion is described as a comparison between both groups, responders and non responders, and thus highly depends on methodological issues such as the sequencing technologies, the size of the cohorts etc... Moreover, each OTU is represented at a different level in relative abundance, some OTU relative abundance ranging from 0.01 to 0.1% of total number of sequences, some other from 0,5% to 35% of the total number of sequences. Preferably, in the method of the invention, OTU relative abundance is assessed by applying a detection threshold of either 0.2%, or preferably 1%, of the total number of sequences (per sample).

The table I below illustrates mean, median, range, 1 st quartile and 3rd quartile of relative abundance of each OTUs (SEQ. ID. N°1 to SEQ. ID. N°16) in each response Group (ie RESPONDERS vs. NON RESPONDERS to a CTLA4 treatment, see the experimental part below). Two criteria have been defined, one taking into account a threshold of relative abundance of the OTU (crit 2), and a second one taking into account detection level of each OTU (crit 1).

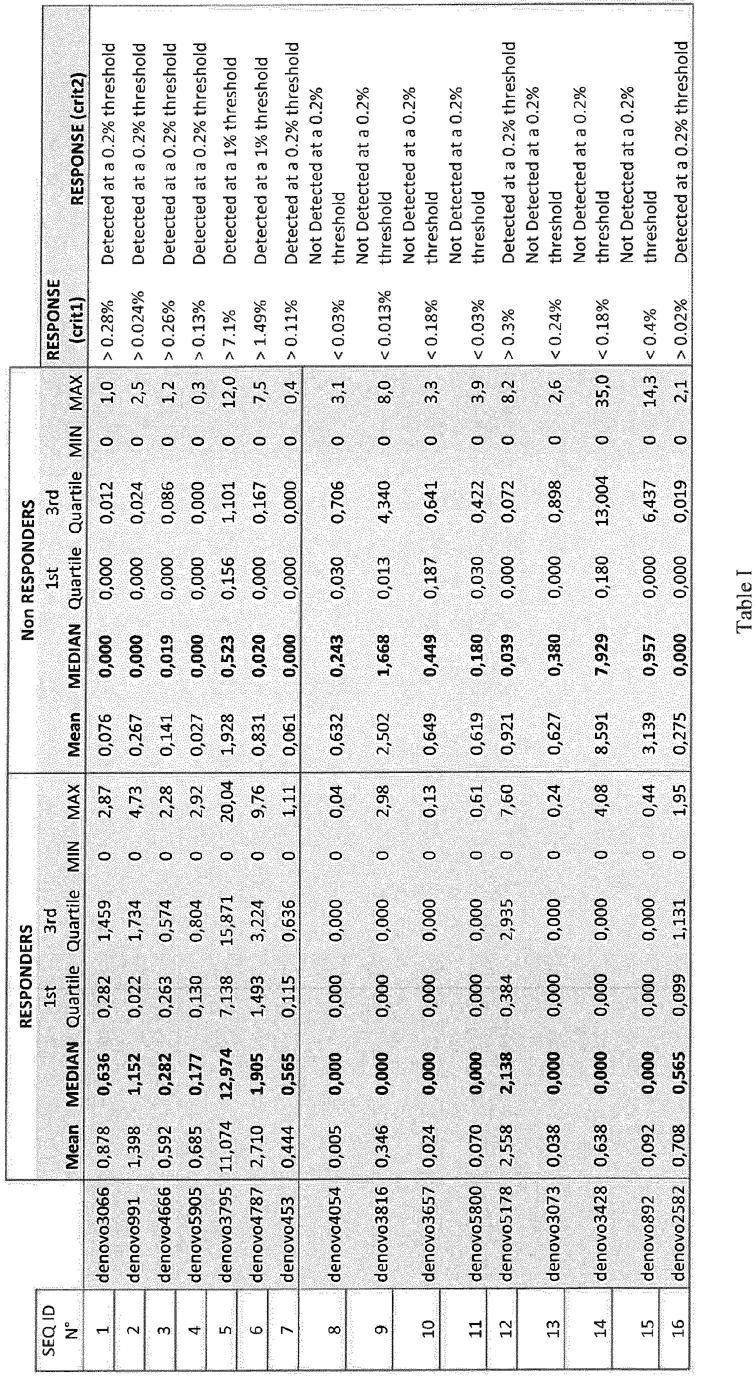

According to another embodiment, the present invention relates to a method for predicting the response of an individual to an anticancer treatment with anti-CTL4 molecules comprising the steps of:
(i) determining gut microbial OTU in a fecal sample of said individual;
(ii) determining the presence or the absence of at least one OTU comprising a nucleotide fragment of sequence having at least 97%, preferably 98%, 99% or 100%, identity with a nucleic sequence selected in the group consisting of: denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7, denovo4054 of SEQ. ID. N°8, denovo3816 of SEQ. ID. N°9, denovo3657 of SEQ. ID. N°10, denovo5800 of SEQ. ID. N°11 and denovo5178 of SEQ. ID. N°12, by applying a detection threshold of 0,2% of the total number of sequences (per sample) to define an OTU's presence or absence, or applying quantitative PCR detection with specific OTU or bacterial isolate targeting probes;
wherein individual having a gut microbiote showing the presence of at least one OTU selected in the group consisting of denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7 and denovo5178 of SEQ. ID. N°12 and/or showing the absence of at least one OTU selected in the group consisting of denovo4054 of SEQ. ID. N°8, denovo3816 of SEQ. ID. N°9, denovo3657 of SEQ. ID. N°10 and denovo5800 of SEQ. ID. N°11 is a good responder to said anticancer treatment.

A patient that is a "good responder to an anticancer treatment" is a patient who is affected with a cancer and who will show a clinically significant response after receiving said anticancer treatment; the clinically significant response may be assessed by clinical examination (body weight, general status, pain and palpable mass, if any), biomarkers and imaging studies (ultrasonography, CT scan, PAT scan, MRI).

As used herein, "cancer" means all types of cancers. In particular, the cancers can be solid or non solid cancers. Non limitative example of cancers are head and neck squamous cell carcinoma, Hodgkin's lymphoma, urothelial cancer of the bladder, mismatch repair deficient colorectal cancer, gastric cancer and merkel cell carcinoma.

The term "treatment" herein refers to any reduction of the progression, severity and/or duration of cancer.

According to a specific embodiment, the individual is a patient with metastatic melanoma.

According to another specific embodiment, anti-CTL4 molecule is an anti-CTL4 antibody, such as ipilimumab.

Accordingly, the method of the invention allows to determine whether a patient with metastatic melanoma is likely to benefit from a treatment with ipilimumab by analysis the gut microbiota in a feces sample from said patient.

Gut microbiote relates to the population of microorganisms living in the intestine of any organism belonging to the animal kingdom; in the present invention, said organism is preferably a human. The gut microbial composition evolves throughout the entire life and is the result of different environmental influences.

Dysbiosis refers to the deleterious loss of balance in gut microbial composition that may arise in some specific situations.

The fecal sample is collected at any moment before deciding the beginning of the anticancer treatment or at any moment during said treatment; preferably, the fecal sample is collected before the start of the anticancer treatment. In any case, it is collected in a patient without colitis.

OTU means operational taxonomic unit; in the present invention, an OTU regroups bacterial 16S sequences sharing the same or similar (at least 97% of sequence identity) and is named "denovo#". Each OTU is affiliated to a bacterial species, either isolated or not, cultured or uncultured when sharing 98% or more sequence similarity with a sequence described in public databases.

In a particular embodiment, step (ii) of the method of the invention consists in determining the relative abundance or the presence or the absence of at least two, three, four, five or six sequences having at least 97%, preferably 98%, 99% or 100%, identity with the nucleic sequence of OTUs selected in the group consisting of: denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7, denovo4054 of SEQ. ID. N°8, denovo3816 of SEQ. ID. N°9, denovo3657 of SEQ. ID. N°10, denovo5800 of SEQ. ID. N°11, denovo5178 of SEQ. ID. N°12, denovo3073 of SEQ. ID. N°13, denovo3428 of SEQ. ID. N°14, denovo892 of SEQ. ID. N°15 and denovo2582 of SEQ. ID. N°16.

Areas under the curves (AUC) have been calculated for several combinations of OTUs to be predictive to the response to anti CTLA4 treatment, the 12 combinations presented in the Table II below had an good predictive value with AUC >0.90:

**Table II**

| **OTUs Combination (SEQ. ID. N°)** | **AUC** |
|---|---|
| 7+8+9+10+11+12 | 0,9 |
| 8+9+10+11+12 | 0,91 |
| 1+6+7+8+9+10 | 0,9215 |
| 1+7+8+9+10 | 0,9215 |
| 1+6+9+10 | 0,9084 |
| 1+7+9+10 | 0,9152 |
| 1+8+9+10 | 0,9215 |
| 1+9+10 | 0,9281 |
| 1+9+10+13 | 0,9215 |
| 1+9+10+14 | 0,9215 |
| 1+9+10+15 | 0,9019 |
| 1+9+10+16 | 0,9215 |

According to one specific embodiment of the method of the invention, step (ii) aims to determine relative abundance (enrichment or depletion) of the above listed OTUs combination; the following relative abundance of said combinations of OTUs in the gut microbiote of an individual means that said individual is a good responder to said anticancer treatment:

| Combination | enrichment | depletion |
|---|---|---|
| A | SEQ. ID. N°: 7, 12 | SEQ. ID. N°: 8, 9, 10, 11 |
| B | SEQ. ID. N°: 12 | SEQ. ID. N°: 8, 9, 10, 11 |
| C | SEQ. ID. N°:1, 6, 7 | SEQ. ID. N°: 8, 9, 10 |
| D | SEQ. ID. N°: 1, 7 | SEQ. ID. N°: 8, 9, 10 |
| E | SEQ. ID. N°: 1,6 | SEQ. ID. N°: 9, 10 |
| F | SEQ. ID. N°: 1, 7 | SEQ. ID. N°: 9, 10 |
| G | SEQ. ID. N°: 1 | SEQ. ID. N°: 8, 9, 10 |
| H | SEQ. ID. N°: 1 | SEQ. ID. N°: 9, 10 |
| I | SEQ. ID. N°: 1 | SEQ. ID. N°: 9, 10, 13 |
| J | SEQ. ID. N°: 1 | SEQ. ID. N°: 9, 10, 14 |
| K | SEQ. ID. N°: 1 | SEQ. ID. N°: 9, 10, 15 |
| L | SEQ. ID. N°: 1, 16 | SEQ. ID. N°: 9, 10 |

According to another specific embodiment of the method of the invention, step (ii) aims to determine the presence of the absence of the above listed OTUs combination; the following results observed for said combinations of OTUs in the gut microbiote of an individual means that said individual is a good responder to said anticancer treatment:

| Combination | presence | absence |
|---|---|---|
| A | SEQ. ID. N°: 7, 12 | SEQ. ID. N°: 8, 9, 10, 11 |
| B | SEQ. ID. N°: 12 | SEQ. ID. N°: 8, 9, 10, 11 |
| C | SEQ. ID. N°:1, 6, 7 | SEQ. ID. N°: 8, 9, 10 |
| D | SEQ. ID. N°: 1, 7 | SEQ. ID. N°: 8, 9, 10 |
| E | SEQ. ID. N°: 1, 6 | SEQ. ID. N°: 9, 10 |
| F | SEQ. ID. N°: 1, 7 | SEQ. ID. N°: 9, 10 |
| G | SEQ. ID. N°: 1 | SEQ. ID. N°: 8, 9, 10 |
| H | SEQ. ID. N°: 1 | SEQ. ID. N°: 9, 10 |
| I | SEQ. ID. N°: 1 | SEQ. ID. N°: 9, 10, 13 |
| J | SEQ. ID. N°: 1 | SEQ. ID. N°: 9, 10, 14 |
| K | SEQ. ID. N°: 1 | SEQ. ID. N°: 9, 10, 15 |
| L | SEQ. ID. N°: 1, 16 | SEQ. ID. N°: 9, 10 |

The Table III below shows the correspondence between OTU and their affiliated bacterial isolate/species :

**Table III**

| OTUs | SEQ. ID. N° | Affiliated bacterial isolate/species | Corresponding bacterial 16S sequence SEQ. ID. N° |
|---|---|---|---|
| denovo3066 | 1 | *Parabacteroides distasonis* | 17 |
| denovo991 | 2 | *uncultured bacterium NA48 AY975552 (Clostridium XIVa*/*Fusicatenibacter saccharivorans)* | 18 |
| denovo4666 | 3 | *butyrate producing bacterium SS2 1 AY305319 (Anaerostipes hadrus)* | 19 |
| denovo5905 | 4 | *uncultured bacterium C3 13 GQ896957 (Ruminococcus*/*Oscillibacter valericigenes)* | 20 |
| denovo3795 | 5 | *Faecalibacterium prausnitzii* | 21 |
| denovo4787 | 6 | *Gemmiger formicilis* | 22 |
| denovo453 | 7 | *uncultured bacterium RL246 aai74e12 DQ793623 (Unclassified Clostridiales*/ *Faecalibacterium sp. canine oral taxon 147)* | 23 |
| denovo4054 | 8 | *Bacteroides ovatus* | 24 |
| denovo3816 | 9 | *Faecalibacterium prausnitzii* | 25 |
| denovo3657 | 10 | *Clostridium sp. XB90* /*Eisenbergiella massiliensis* | 26 |
| denovo5800 | 11 | *uncultured bacterium REC1M 21 AY343160 (Oscillibacter sp. Marseille-P2778)* | 27 |
| denovo5178 | 12 | *butyrate producing bacterium L2 21 AJ270477* / *Eubacterium rectale* | 28 |
| denovo3073 | 13 | *Bacteroides uniformis JCM5828 3 EU136680* | 29 |
| denovo3428 | 14 | *Bacteroides sp ALA Bac AM117579* | 30 |
| denovo892 | 15 | *Bacteroides uniformis JCM 5828T AB050110* | 31 |
| denovo2582 | 16 | *Blautia obeum 1 33 AY169419* / *Blautia wexlerae JCM17041* | 32 |

According to a further embodiment, step (ii) of the method of the invention consists in detecting the relative abundance and/or the presence or absence of at least one of the bacterial isolate/species affiliated to the OTU of SEQ. ID. N°1 to 12 by culturing a feces sample on appropriate culture medium and in appropriate culture conditions,
wherein enrichment or presence in an individual's gut microbiote of bacterial isolate/species selected in the group consisting of *Parabacteroides distasonis, uncultured bacterium NA48 AY975552 (Clostridium XIVa*/ *Fusicatenibacter saccharivorans), butyrate producing bacterium SS2 1 AY305319 (Anaerostipes hadrus), uncultured bacterium C3 13 GQ896957 (Ruminococcus*/*Oscillibacter valericigenes), Faecalibacterium prausnitzii, Gemmiger formicilis, uncultured bacterium RL246 aai74e12 DQ793623 (Unclassified Clostridiales*/*Faecalibacterium sp. canine oral taxon* 147) and *butyrate producing bacterium L2 21 AJ270477* / *Eubacterium rectale* and/or the depletion or absence in said individual's gut microbiote of bacterial isolate/species selected in the group consisting of *Bacteroides ovatus, Faecalibacterium prausnitzii, Clostridium sp. XB90* / *Eisenbergiella massiliensis and uncultured bacterium REC1M 21 AY343160 (Oscillibacter sp. Marseille-P2778)* means that said individual is a good responder to said anticancer treatment.

According to another embodiment, step (ii) of the method according to the present invention consists in determining the relative abundance of at least one OTU comprising a nucleic acid fragment of 300 to 1500 pb having a sequence of at least 97%, preferably 98%, 99% or 100%, identity with a nucleic acid fragment sequence of a 16S rRNA selected in the group consisting of: SEQ. ID. N°16, SEQ. ID. N°17, SEQ. ID. N°18, SEQ. ID. N°19, SEQ. ID. N°20, SEQ. ID. N°21, SEQ. ID. N°22, SEQ. ID. N°23, SEQ. ID. N°24, SEQ. ID. N°25, SEQ. ID. N°26, SEQ. ID. N°27 and SEQ. ID. N°28.

From the OTUs population described in the overall cohort, metabolomic profiles have been deduced *in silico* applying the PICRUSt algorithm combined with LEFSe.

A specific set of metabolic pathways (described as KEGG IDs) have been highlighted specifically associated with response to anti CTLA4 or toxicity to anti CTLA4 (see experimental part IV).

To further validate this *in silico* obtained results and/or highlight new metabolites, both known or unknown, that would discriminate patients for their response to antiCTLA4, metabolomic profiling of the 26 patients' feces at baseline is currently under evaluation.

The present invention thus also relates to a method for predicting the response of an individual to an anticancer treatment with anti-CTL4 molecules comprising the step of determining the presence or the absence of at least one metabolite (described as KEGG ID) selected in the group consisting of:
Membrane and intracellular structural molecules
Other glycan degradation
Lipopolysaccharide biosynthesis
Other ion_coupled transporters
Aminosugar and nucleotidesugar metabolism
Carbon fixation pathways in prokaryotes
Purine metabolism
Galactose metabolism
Sphingolipid metabolism
Chaperones and folding catalysts
Fructose and mannose metabolism
Lysosome
Oxidative phosphorylation
Alanine_aspartate and glutamate metabolism
Peptidases
Secretion system
Porphyrin and chlorophyll metabolism
Two_component system
Flagellar assembly
Bacterial chemotaxis
Bacterial motility proteins
ABC transporters
Transporters
in a biological sample of said individual, wherein the presence of at least one metabolite selected in the group consisting of:
Secretion system
Porphyrin and chlorophyll metabolism
Two_component system
Flagellar assembly
Bacterial chemotaxis
Bacterial motility proteins
ABC transporters
Transporters
is associated with a good response to said anti-cancer treatment and/or the presence of at least one metabolite selected in the group consisting of:
Membrane and intracellular structural molecules
Other glycan degradation
Lipopolysaccharide biosynthesis
Other ion_coupled transporters
Aminosugar and nucleotidesugar metabolism
Carbon fixation pathways in prokaryotes
Purine metabolism
Galactose metabolism
Sphingolipid metabolism
Chaperones and folding catalysts
Fructose and mannose metabolism
Lysosome
Oxidative phosphorylation
Alanine_aspartate and glutamate metabolism
Peptidases
is associated with a poor response to said anti-cancer treatment.

### FIGURES LEGENDS

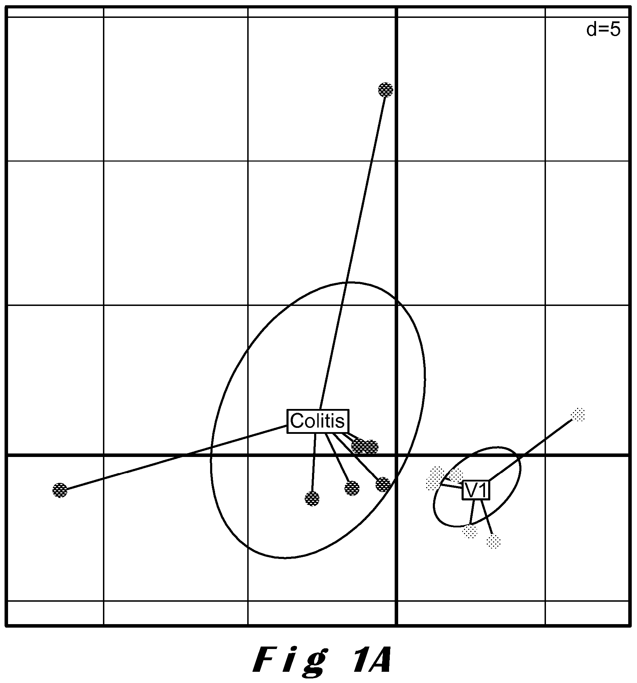

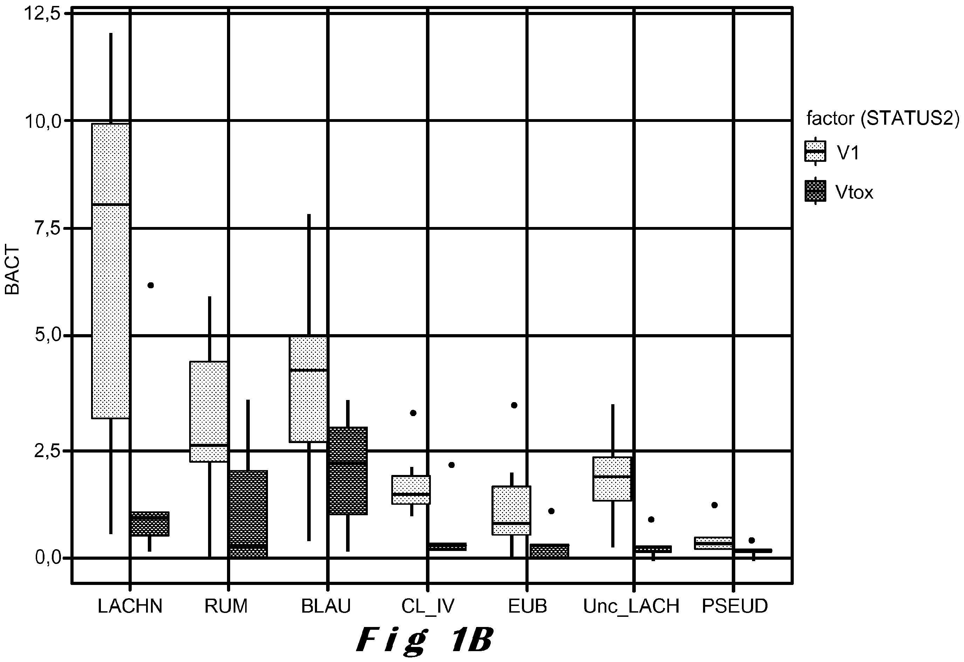

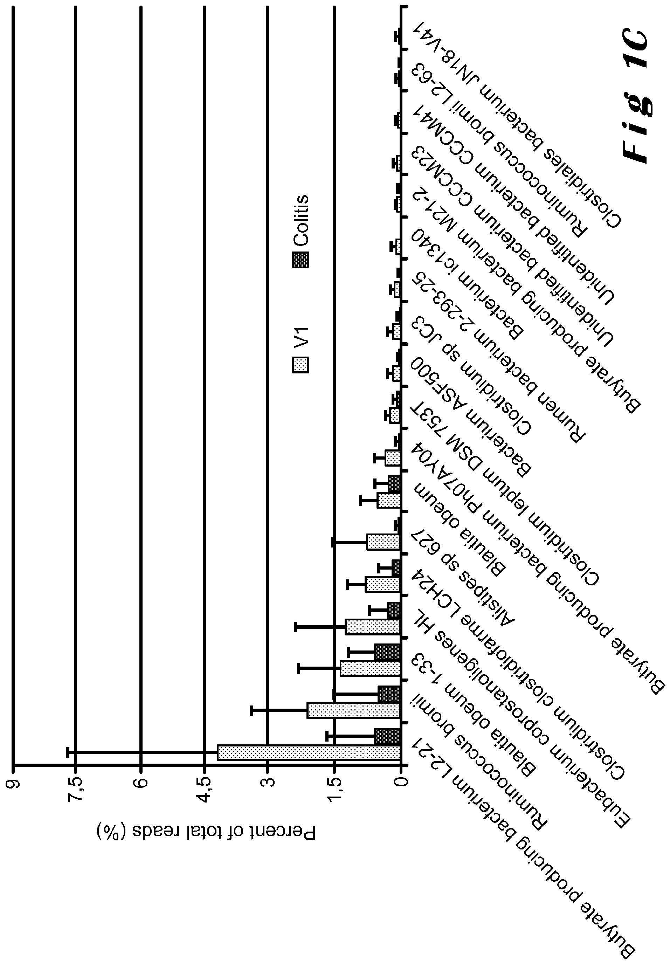

**Figure 1****. Gut microbiota at baseline and during colitis in patients with metastatic melanoma treated with ipilimumab.**
   **A.** Principal component analysis representation of patient distribution based on bacterial genera composition between baseline visit (V₁) and colitis. Seven patients had fecal samples collected at both baseline and time of colitis onset. Monte-Carlo simulated p-value= 0.0059. Component 1 explains 13.59 % of variance; component 2 explains 11.45% of variance.
   **B.** Relative abundance of dominant (>1% of total reads) gut microbial genera significantly reduced during colitis (Vₜₒₓ) as compared to baseline (V₁). LACHN: *Lachnospiracea incertae sedis,* RUM: *Ruminococcus,* BLAU: *Blautia,* CL_IV: *Clostridium* IV, EUB: *Eubacterium,* Unc_LACH: unclassified *Lachnospiraceae,* PSEUD: *Pseudoflavonifractor* (paired t-test p<0.05 for all genera). **C.** Proportions as percent of total reads of significantly impacted bacterial isolates during colitis (Vₜₒₓ) as compared to baseline (V₁). Only significant (paired t-test p<0.05) data are presented.

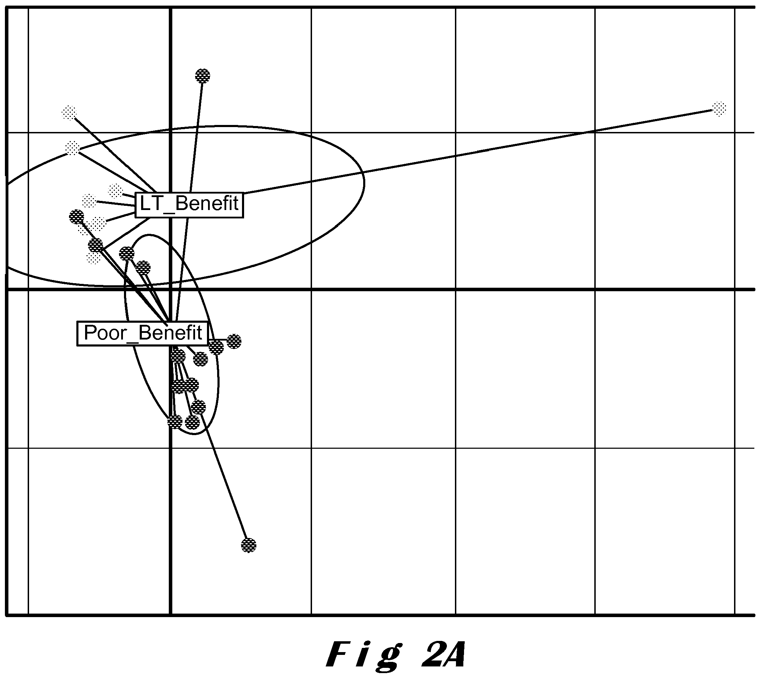

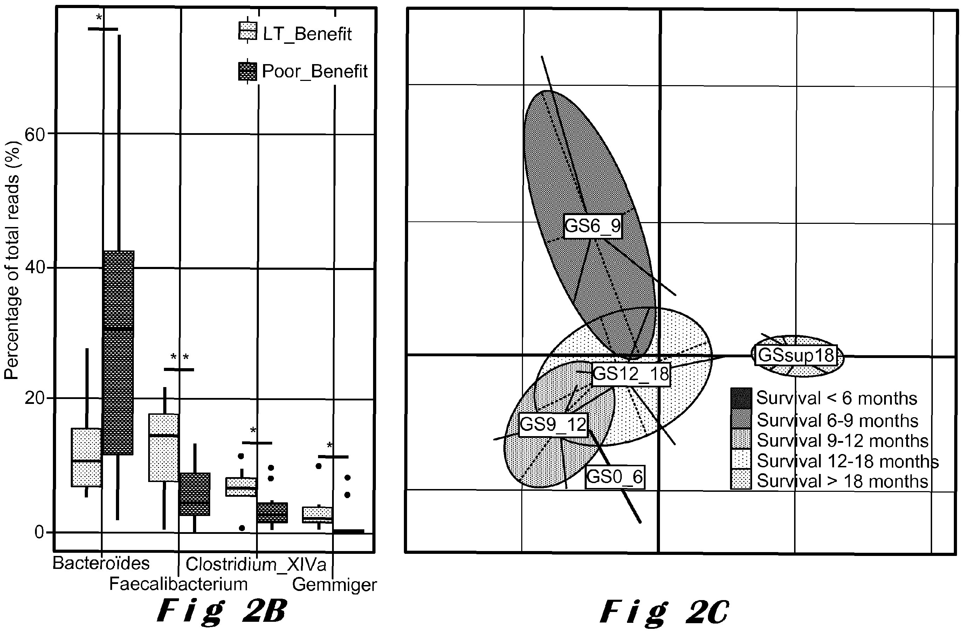

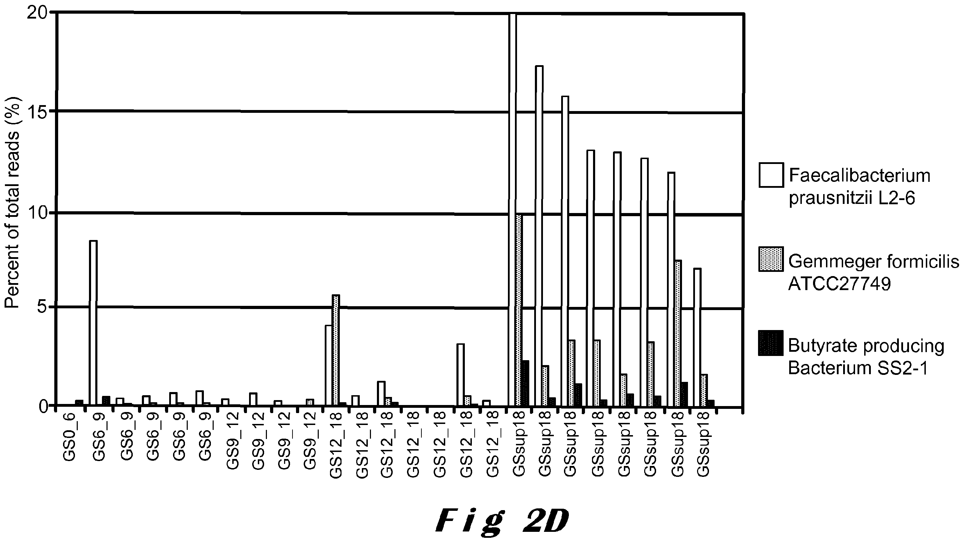

**Figure 2****. Baseline gut microbiota as a predictor of response to ipilimumab**
   **A.** Principal component analysis representation of patients' distribution based on bacterial genera composition at baseline (V₁) depending on the benefit of ipilimumab treatment; LT Benefit: long-term benefit (in green) vs Poor Benefit (in dark red). Component 1 explains 12.86 % of variance; component 2 explains 8.67% of variance. Monte-Carlo simulated p-value=0.00899.
   **B.** Boxplot of the percentages of 4 dominant (>1% of total reads) genera differentially represented between both groups, i.e. *Bacteroides, Faecalibacterium, Clostridium* XIVa and *Gemmiger* ; LT_Benefit: long-term benefit vs Poor Benefit; *: p<0.05; **: p<0.001.
   **C.** Inter-class principal component analysis representation of patient distribution based on bacterial genera composition at baseline (V₁) depending on overall survival time following ipilimumab treatment; GS0_6: overall survival ranging from 0 to 6 months (n=2), GS6_9: overall survival ranging from 6 to 9 months (n=5), GS9_12: overall survival ranging from 9 to 12 months (n=4), GS12_18: overall survival ranging from 12 to 18 months (n=7), GSsup18: overall survival superior to 18 months (n=8). Monte-Carlo simulated p-value= 0.01098.
   **D.** Percentages of 3 specific OTUs highlighted as biomarkers at baseline (V₁) of overall survival duration greater than 18 months. Each patient's microbiota are presented in the graph. GS0_6: overall survival ranging from 0 to 6 months (n=2), GS6_9: overall survival ranging from 6 to 9 months (n=5), GS9_12: overall survival ranging from 9 to 12 months (n=4), GS12_18: overall survival ranging from 12 to 18 months (n=7), GSsup18: overall survival greater than 18 months (n=8).

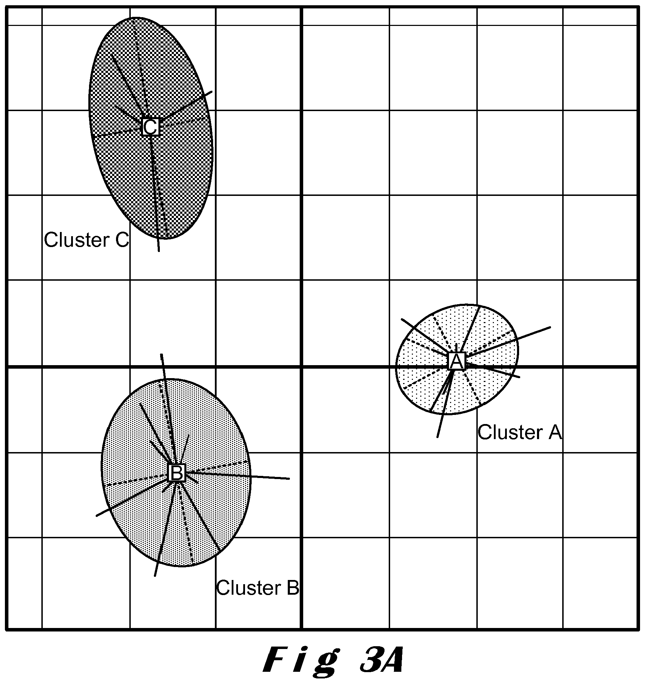

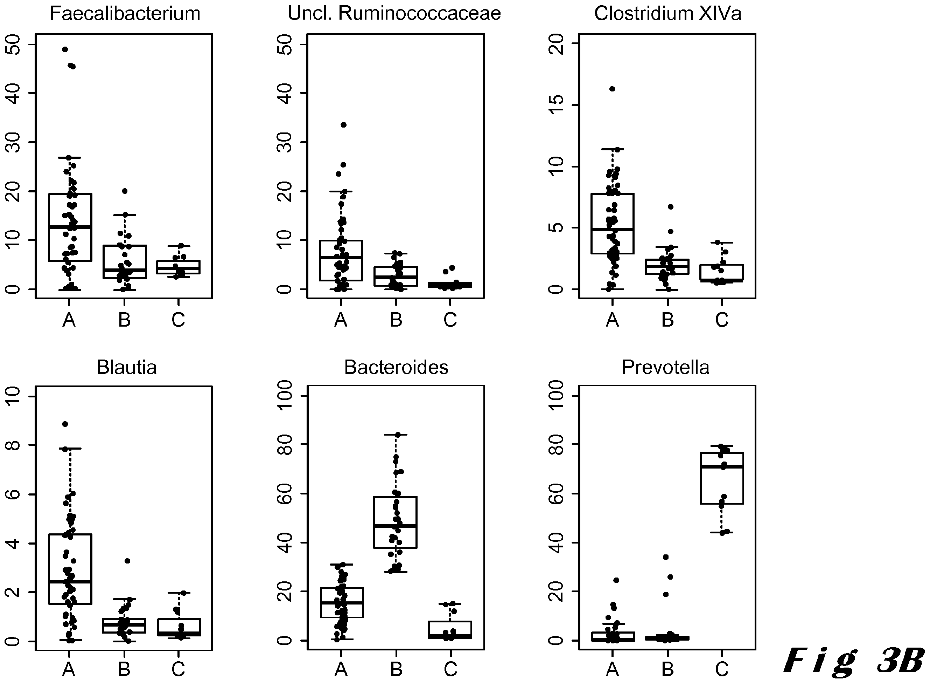

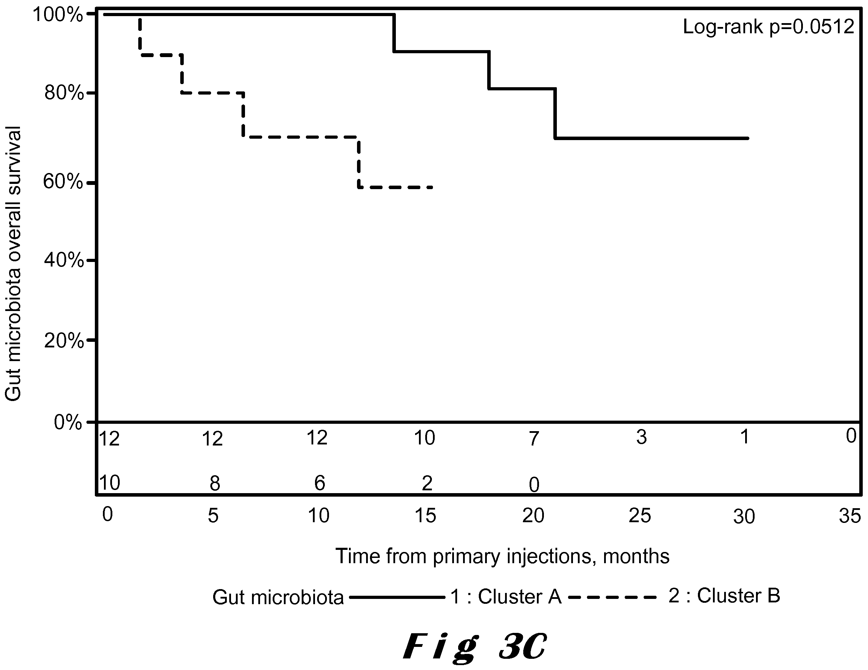

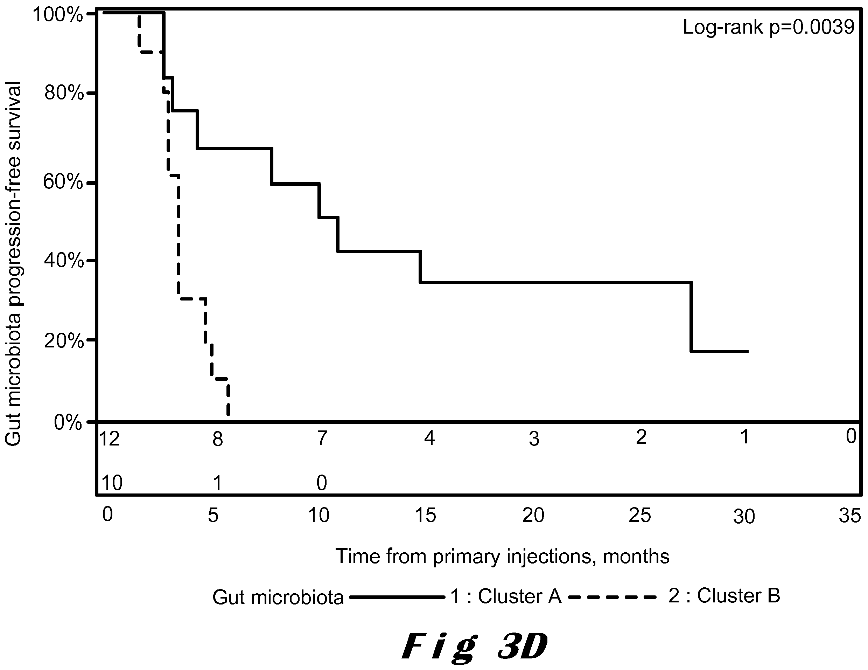

**Figure 3****. Baseline Gut microbiota composition predicts clinical response to ipilimumab.**
   **A.** Inter-class principal component analysis representation of patient's stratification into 3 statistically robust clusters (A, B and C) at baseline (V₁). Monte-Carlo simulated p-value=0.00099.
   **B.** Bacterial genera discriminating the 3 different clusters at baseline were assessed with a Random Forest analysis, and confirmed with a Wilcoxon test. Percentage of reads for each of these 6 genera is represented for each cluster. Genera significantly (Wilcoxon test) overrepresented in patients from Cluster A are in blue, genus significantly overrepresented in patients from Cluster B is in green, and genus significantly overrepresented in patients from Cluster C is in grey.
   **C.** Baseline gut microbiota composition and overall survival (OS). Kaplan-Meier survival curves of patients classified into two groups according to clusters, Cluster A *versus* Cluster B.
   **D.** Baseline gut microbiota composition and progression free survival. Kaplan-Meier curves of patients classified into two groups according to clusters, Cluster A versus Cluster B. P values are indicated on each graph.

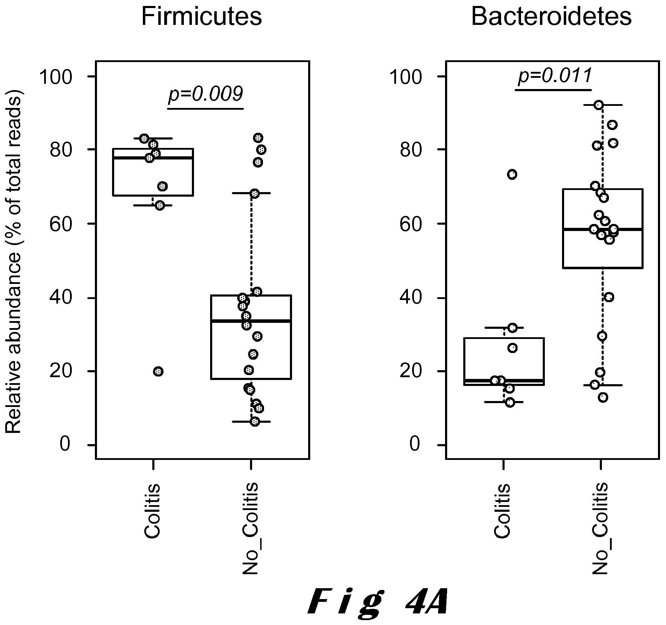

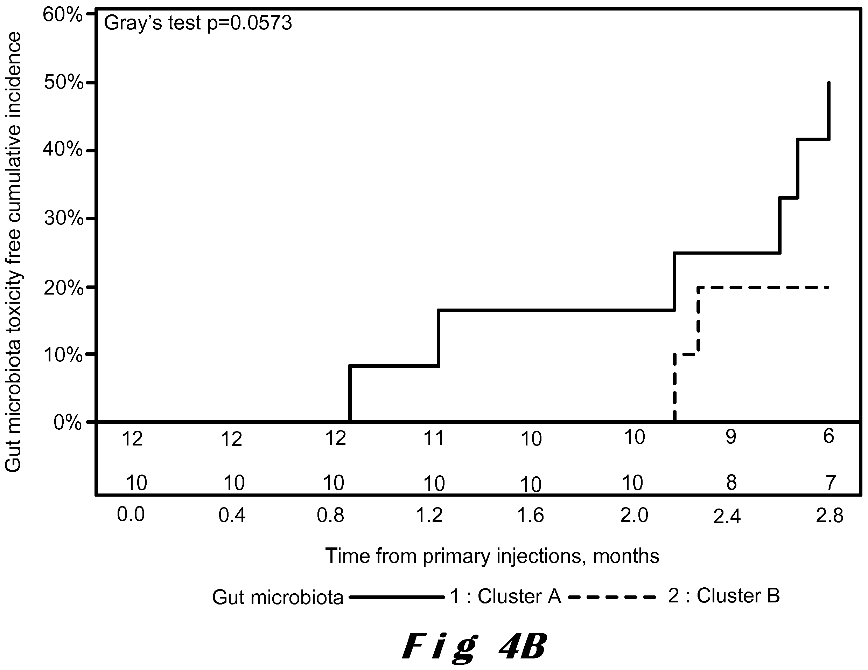

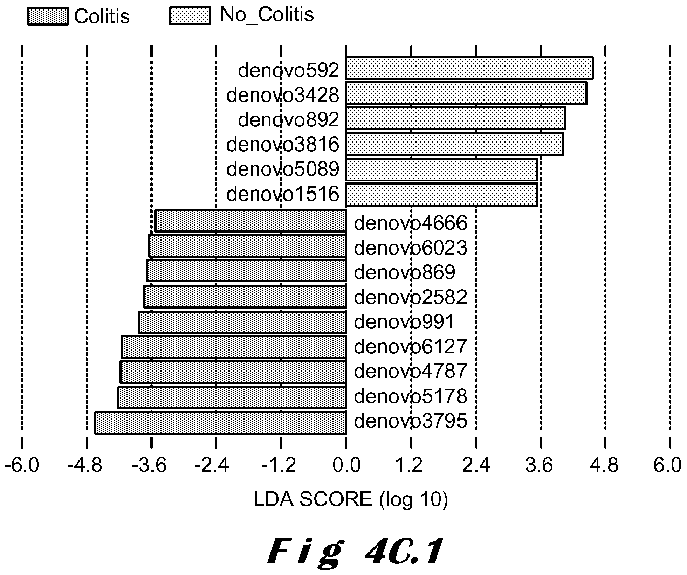

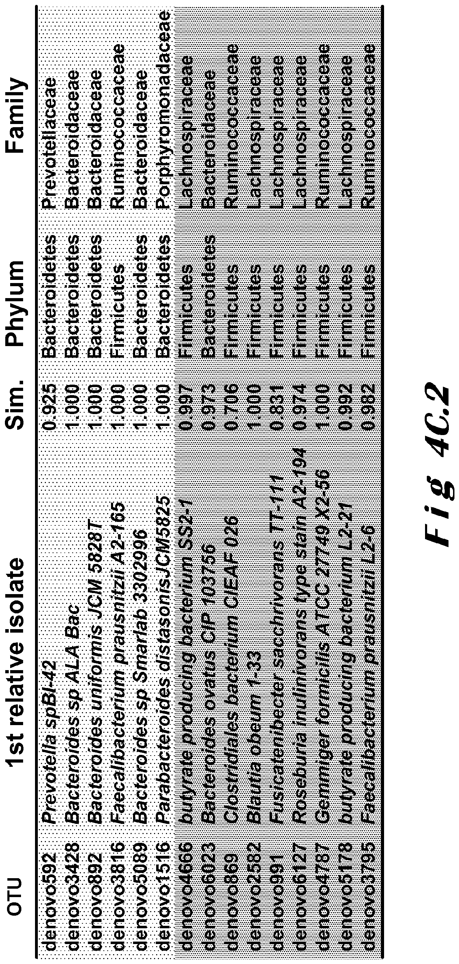

**Figure 4****. Gut microbiota composition at baseline predicts ipilimumab-induced colitis.**
   **A.** Boxplot of relative abundance of the 2 dominant phyla Firmicutes and Bacteroidetes at baseline between patients prone to or resistant to ipilimumab-induced colitis. A Wilcoxon test has been applied to assess significance and p-values are indicated on the graph.
   **B.** Colitis cumulative incidence (Gray's test) of patients classified into two groups according to clusters, Cluster A versus Cluster B. P values are indicated on the graph.
   **C.** OTUs predictive of colitis development during ipilimumab treatment. LEfSe uses Linear Discriminant Analysis (LDA) to estimate the effect size of each differentially abundant feature (i.e. bacterial OTU). On the left panel, OTUs in red are biomarkers of colitis development (Colitis). OTUs in green are biomarkers of the absence of colitis development (No_Colitis). Best biomarkers show the highest absolute LDA score with a minimal threshold of 3.5 (i.e. OTU denovo592 for the No_Colitis group and OTU denovo3795 for the Colitis group). Right panel indicates the taxonomic affiliation of each of these OTUs. Sim.: similarity between the OTU read and the first assigned isolate 16S sequence, assessed by the RDP Sab_score. The RDP S_ab score is a percentage of shared 7-mers between two sequences.

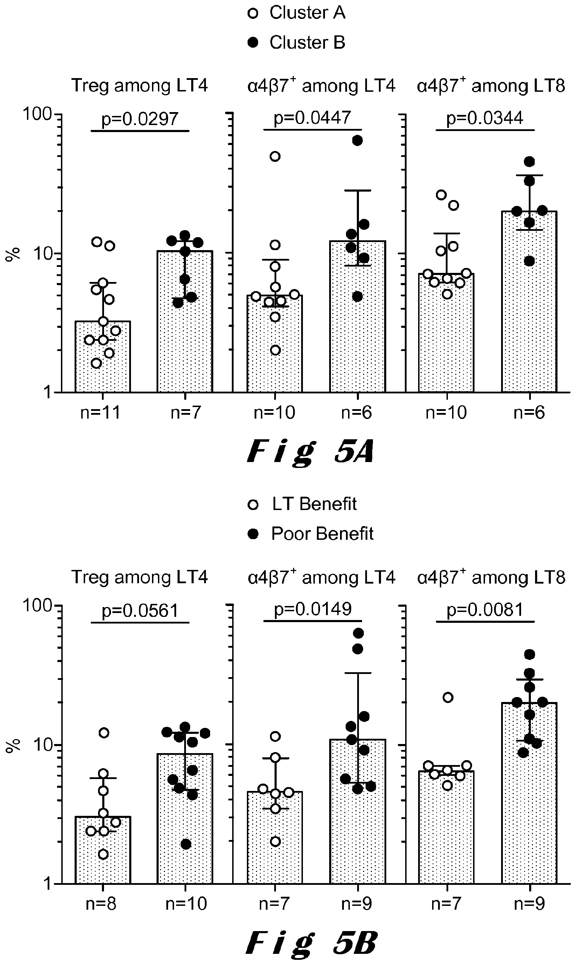

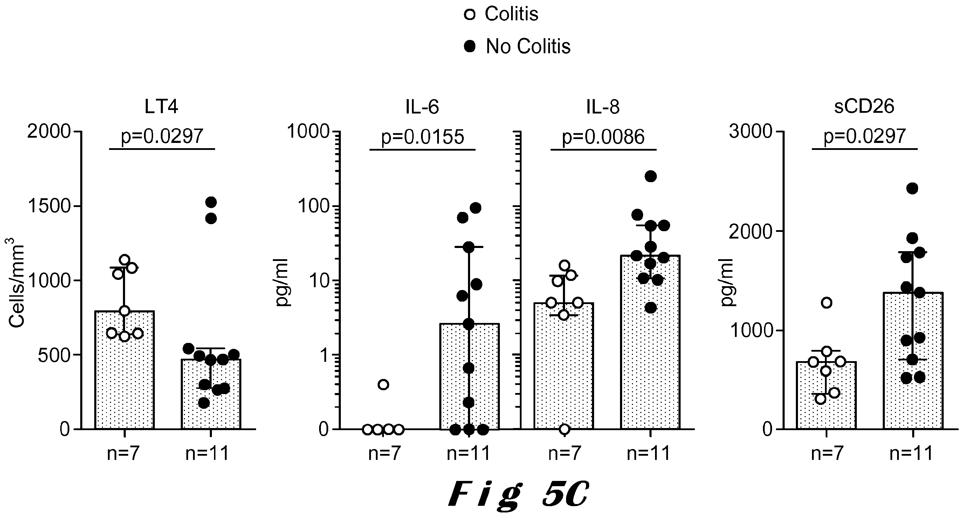

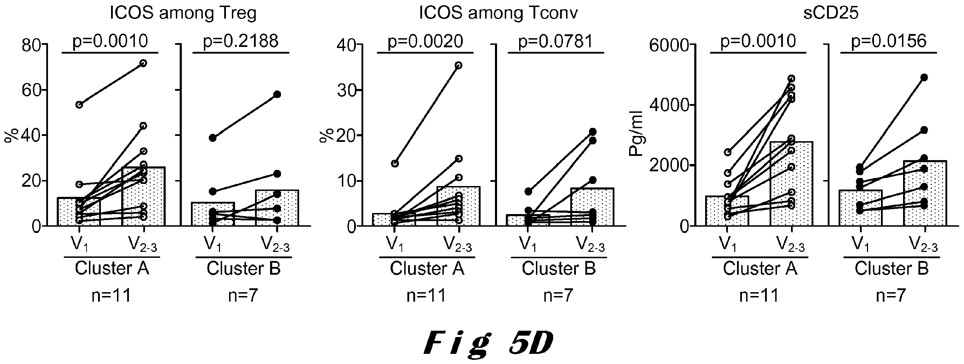

**Figure 5****. Systemic immune status and microbiota composition**
   **A-C.** Baseline (V₁) percentages and absolute numbers of fresh whole blood CD4⁺ T cells, Treg cells (% CD4⁺CD3⁺CD25⁺Foxp3⁺ within CD4⁺CD3⁺ cells) ; α4⁺β7⁺ among CD4⁺ T and CD8⁺ T cells, serum concentrations of IL-6, IL-8 and sCD25 were analyzed and compared between patients belonging to Cluster A and patients belonging to Cluster B **(A);** long term clinical benefit (LT benefit) and poor clinical benefit (Poor benefit) **(B);** patients with colitis (Colitis) and patients without colitis (No colitis) **(C).** Each dot represents one patient. P values are indicated on each graph; ns means "not significant"; Mann Whitney tests were used.
   (D) The expression of ICOS on CD4+ 21 T cells was monitored in fresh whole blood before ipilimumab treatment (V₁) and after one or two injections of ipilimumab (V2-3). Serum concentration of sCD25 was monitored prior to ipilimumab treatment (V1) and after one or two injections of ipilimumab (V2-3). Percentage of conventional CD4+ 24 T cells (Tconv) was defined as %CD3+CD4+ T excluding Treg cells. Graphs depict specific changes in immune activation over the course of ipilimumab treatment in patients belonging to Cluster A (white circles) and patients belonging to Cluster B (black circles). Each dot represents one patient. 1 P values are indicated on each graph. Mann Whitney (A-C) and Wilcoxon matched-pairs signed rank (D) tests were used.

### EXAMPLES

### I. PATIENTS, MATERIALS AND METHODS

### Patients

Patients with MM treated with ipilimumab were prospectively enrolled at the Gustave Roussy Cancer Campus between March 2013 and December 2014. Patients were informed of the study and consented to participate. This study was approved by the Kremlin Bicêtre Hospital Ethics Committee (GOLD study: SC12-018; ID-RCB-2012-A01496-37) and all procedures were performed in accordance with the Declaration of Helsinki. Patients had a pre-specified clinical workup; feces and blood were collected at baseline (V₁), prior to each ipilimumab infusion (V₂, V₃, V₄), at the end of treatment (ie, 3 weeks after the last infusion; V₅) and, if present, at the time of colitis occurrence (V_{Tox}). Ipilimumab was administered intravenously every 3 weeks at a dose of 3 or 10 mg/Kg and could be continued after V₄, at a maintenance dose of one infusion every 12 weeks, in patients whose disease was controlled (response or stable disease). When immune-mediated enterocolitis (≥ grade III) was suspected, patients were referred to the Gastroenterology Department of Bicêtre Hospital. The diagnosis of ipilimumab-induced colitis was made in patients who had endoscopic signs of inflammation and no other cause of colitis, such as ischemia and infection (stool tests for bacterial pathogens and *Clostridium difficile* toxin had to be negative).

Response to ipilimumab was assessed by several criteria. Long-term clinical benefit was defined by response decrease of tumour burden ≥ 50% relative to baseline, according to immune related response criteria [15, 16]) or stable disease (decrease of tumour burden of less than 50% but with less than 25% increase relative to nadir) for more than 6 months. Patients with poor benefit were defined as patients with a lack of long term benefit, i.e. with progression-free survival of less than 6 months (with immune related progressive disease defined as a confirmed increase in tumor burden ≥ 25% relative to nadir) [15, 16]. All responses were confirmed by a subsequent assessment no less than 4 weeks from the date first documented.

### Bacterial composition assessment by high-throughput sequencing

Fecal samples were collected anaerobically at baseline (V₁), prior to each ipilimumab infusion (V₂, V₃, V₄), at the end of the treatment (V₅) and at the time of colitis (V_{Tox}) and were kept at -80°C until analysis. Total DNA was extracted from fecal sample aliquots (∼150mg), as previously described using both physical and chemical lysis [17]. Culture-independent 16S rRNA gene sequencing was performed on 83 fecal samples (*n*=26 patients; Table S3). Both 454 pyrosequencing (Life Sciences, a Roche company, Branford, CT, USA) and MiSeq (Illumina Inc, San Diego, CA, USA) technologies were applied on the V3-V4 16S rRNA gene region. Gut bacterial composition was determined prospectively without knowledge of colitis or of clinical benefit, which was determined at the end of the study.

More precisely, for both pyrosequencing (454) and MiSeq sequencing, the V3-V4 region of the 16S rRNA gene was amplified with the following primers: V3F «TACGGRAGGCAGCAG» (V3F bac339F modified with R instead of G) (Wilson KH, et al. J Clin Microbiol. 1990) and V4R «GGACTACCAGGGTATCTAAT» bac806R. For 454, applied quality filters were minimum length=250 bp, maximum length=600 bp, minimum quality threshold= 25, maximum number of homopolymers=6. For MiSeq, the filters were minimum length=200 bp and minimum quality threshold= 20.

Applying these filters, an average of 1639 and 7377 reads/samples were kept for further analysis respectively with 454 and MiSeq technology. High quality reads were pooled, checked for chimeras, and grouped into Operational Taxonomic Units (OTUs) based on a 97% similarity threshold with uclust software from QIIME. Finally 462,312 high-quality reads (average 4,623 reads/sample) were analyzed and grouped into an average of 349 Operational Taxonomic Units (OTUs) per sample (97% similarity threshold; OTUs or phylotypes) based on uclust software from QIIME [1]. Estimates of phylotypes richness and diversity were calculated using both Shannon and Simpson indices on the rarefied OTU table (*n*=1,000 reads). Singletons were removed and phylogenetic affiliation of each OTU was done by using ribosomal database project taxonomy [2] and performed from phylum to species level.

The applied sequencing technology is indicated for each sample (*n*=83) in the Table S3, as is the number of samples used for comparisons within the different part of the study. Statistical analyses were applied to decipher the influence of ipilimumab treatment, colitis development, and other clinical factors on the microbiota and to highlight a combination of microbial groups and immunological defects significantly involved in colitis development in MM patients.

Statistical testing for significant differences between all combinations of two groups was conducted using either paired Student or the Mann-Whitney test. The statistical 1 language R was used for data visualization and to perform abundance-based principal component analysis (PCA) and inter-class PCA associated with Monte-Carlo rank testing on the bacterial genera.

The Random Forest classifier was applied to relative abundance data of distributed bacterial genera and OTUs to assess the variable importance of microbial components in the classification of each patient to a certain phenotype [3].

The random forest machine learning analyses were applied to identify bacterial genera level that differentiates fecal community composition between groups. The purpose of a classifier such as random forest is to learn a function that maps a set of input values or predictors (here, relative to genera abundances) to a discrete output value (here, relative to clinical groups).

Random forest is a powerful classifier that can use nonlinear relationships and complex dependencies between taxa. The degree of the success of the method is its ability to classify unseen samples correctly, estimated by training it on a subset of samples, and using it to categorize the remaining samples [3]. The cross-validation error is compared with the baseline error that would be achieved by always predicting the most common category. Random forest analysis was performed for each comparison on 5000 rarefied versions of the data.

Additionally, random forest assigns an importance score to each genus by estimating the increase in error caused by eliminating that genus from the set of predictors. It also reports the Gini importance of a variable, which is computed as the sum of the Gini impurity decrease of every node in the forest for which that variable was used for splitting. Moreover, the LEfSe (linear discriminant analysis effect size) algorithm [4] was applied for high dimensional discovery of biomarkers that discriminate between patients that will have colitis but who may also respond (or not) to ipilimumab. The algorithm is freely available at http://huttenhower.sph.harvard.edu/galaxy/. Finally, patients were statistically stratified based on their dominant gut microbial composition (genus level). Clustering of patients was performed with the k-means clustering algorithm and Calinski-Harabasz index 1 to select the optimal number of clusters as previously described [5]. The k-means clustering algorithm implemented in R package was used. Interclass PCA of genera composition with clusters as instrumental variable was also assessed, based on a Monte Carlo test with 1000 replicates.

Lastly, random forest analysis was also performed to identify bacterial genera that differentiated fecal community composition between the three identified clusters.

### Fresh Whole Blood Immune monitoring and soluble immune markers

Blood samples were collected at baseline (V₁), prior to each ipilimumab infusion (V₂, V₃, V₄), at the end of treatment (V₅) and at the time of colitis (V_{Tox}). Phenotyping was performed on fresh whole blood or on fresh peripheral blood mononuclear cells isolated by Ficoll density gradient and frozen for later analyses (see supplementary materials). All serum samples were stored at -80°C until further analysis of soluble immune markers of inflammation (IL-6, IL-8, IP-10, MCP-1, TNFα, sCD25) and bacterial translocation (sCD14). Details of immune monitoring can be found in supplementary materials. We monitored memory T cells and ICOS induction on T cells because previous studies had demonstrated that both could be related to clinical responses [18]. Regulatory T cells play a crucial role in the maintenance of immune homeostasis in the gut and are supposed to be a major target for anti-CTLA-4 treatment [19]. The heterodimer α4β7 plays a crucial role in the intestinal homing of T cells; we monitored α4⁺β7⁺ T cells to gain some insight in the mechanism of ipilimumab-induced colitis. We monitored soluble immune markers of inflammation and bacterial translocation as a complement to microbiota composition. Indeed, microbiota composition affects gut barrier function, local as well as systemic immunity [20-25] [26]

### Statistical analyses

A formal sample-size calculation was not performed for this pioneering study. Associations between microbiota dominant profile and immunological parameters were assessed with the Spearman correlation coefficient and a two-sided Wilcoxon test. No adjustment for multiple comparisons was made because of the exploratory component of the analyses. Fisher's exact test was used to examine the association between microbiota clusters and immune parameters; microbiota clusters and colitis, as well as microbiota clusters and clinical responses.

Overall survival (OS) and progression-free survival (PFS) according to intestinal microbiota (Cluster A *vs*. Cluster B) and according to the number of conventional CD4⁺ T cells (low number of conventional CD4 vs. high number of conventional CD4) were estimated using the Kaplan-Meier method and compared using the log-rank test.

When considering the occurrence of toxicity, progression and death lead to informative censoring, because when patients are censored at progression or death, their risk of toxicity could differ from that of patients who are not censored. Competing risk analysis is a method to deal with competing events, in order to avoid selection bias induced by informative censoring.

Therefore, toxicity cumulative incidence function (CIF) was estimated through a competing risk analysis in which toxicity was the event of interest, death or progression was the competing event and patients alive without progression or toxicity at their last follow-up were censored from the date of last follow-up. The toxicity cumulative incidence functions in the different gut microbiota clusters and for the different conventional CD4 levels were estimated using the SAS macro %CIF and compared using the stratified Gray's test [27, 28]. These analyses were performed using SAS software, Version 9.4 (SAS Institute Inc., Cary, NC, USA).

### II. RESULTS

### Clinical characteristics of the patients

Fifty-five patients were included in the study and followed-up for at least 6 months. Among them, 13 patients did not receive ipilimumab, while four patients received only a single infusion of ipilimumab and died soon thereafter due to their melanoma; they were therefore excluded from further analyses. Among the 38 remaining patients, 26 provided fecal samples at baseline (i.e. before the first dose of ipilimumab) for microbiota analysis. Nine of these 26 patients (34.6 %) had a long-term clinical benefit and seven (26.9%) developed an immune-mediated colitis.

### Microbiota composition is not modified by ipilimumab treatment, except at the time of colitis

The gut microbiota was not significantly modified over the course of the ipilimumab treatment. Main bacterial phyla, *Firmicutes* and *Bacteroidetes,* remained stable over time. Even though bacterial genera proportions differed between the different time points, they were not significantly altered by ipilimumab treatment (Wilcoxon test p>0.05). Likewise, diversity was not altered by ipilimumab injections as assessed by both Shannon and Simpson indices. Altogether these data indicate that ipilimumab does not induce a gut microbial dysbiosis in patients with MM.

Seven patients provided fecal samples both at baseline (V₁) and during colitis (Vₜₒₓ). Immune-related colitis was associated with a shift in the gut microbial composition as highlighted on the principal component analysis (PCA; **Fig. 1A****).** These differences were significant at the family level (Wilcoxon test, *p*=0.0049) and at the genus level (*p*=0.0059). The proportions of seven genera were significantly reduced by at least 2 fold at Vₜₒₓ, compared to V₁ **(****Fig. 1B**). They all belonged to the *Firmicutes* phylum (*Ruminococcus, Lachnospiracea incertae sedis, Blautia, Clostridium IV, Eubacterium,* unclassified *Lachnospiraceae* and *Pseudoflavonifractor*) and were dominant members of the microbiota. Similarly, 18 bacterial isolates, mostly *Firmicutes,* were significantly reduced between V₁ and Vₜₒₓ (paired Student t-test p<0.05, **Fig. 1C**). Finally, colitis was associated with a decreased bacterial diversity, as reflected by a lower Shannon diversity index at the time of colitis (V_{1 Shannon}=5.58, V_{tox Shannon}=4.65; paired student t-test *p*=0.042).

### Baseline microbiota composition is associated with subsequent clinical response and immune-related colitis

At V₁, inter-class PCA based on genera composition showed a significant clustering of patients depending on their clinical benefit in response to ipilimumab, either long-term clinical benefit or poor clinical benefit (Monte-Carlo test, *p*=0.00899; **Fig. 2A**). This was also true at finer taxonomic levels such as species or OTUs composition (*p*=0.00499 and *p*=0.00799 respectively). Moreover, fecal microbiota at V₁ allowed the correct classification of 84.6% of patients into either the long-term or poor clinical benefit category, and the accuracy reached more than 99% (5,000 bootstraps) for patients with poor benefit, as assessed by a Random Forest analysis. The receiver operating characteristic (ROC) curve provided an area under the curve (AUC) of 0.77, which indicates a fair classification based on overall microbial composition. Main genera accounting for this stratification are *Faecalibacterium, Gemmiger, Bacteroides* and *Clostridium* XIVa, and when proportions of these genera were solely considered for testing the stratification the classification power increased and the score was nearly excellent (AUC=0.895). While high proportions of *Bacteroides* were present at baseline in patients with poor clinical benefit (Wilcoxon test, *p*=0.03363), *Faecalibacterium* percentages were significantly higher in patients with long-term benefit (*p*=0.009248; **Fig. 2B****).** Baseline fecal microbiota composition was also significantly associated with patients' overall survival and survival longer than 18 months (Wilcoxon test, *p*=0.011; **Fig. 2C****).** All patients with an overall survival longer than 18 months could indeed be classified based on their gut microbial composition at V₁ and all showed higher proportions of OTUs affiliated to Firmicutes: *Ruminococcus* and *Lachnospiraceae* genus (relatives of *Faecalibacterium prausnitzii* L2-6, *Gemmiger formicilis* and *butyrate-producing bacterium* SS2-1; **Fig. 2D****).** It should be noted that although prescription of antibiotics may alter microbiota composition, antibiotics prior to ipilimumab treatment does not influence baseline dominant microbiota and none of the potentially predictive OTUs or bacterial taxon were associated with antibiotic use.

When the microbiota composition of patients was considered at baseline independently from their clinical characteristics and without *a priori* hypotheses (k-means clustering algorithm and Calinski-Harabasz index), three groups of patients emerged that were defined as clusters: Cluster A, B and C. The inter-class PCA based on genera composition depending on belonging to any of these 3 clusters highlighted that this stratification was significant (Monte-Carlo test, *p*=0.00099; **Fig. 3A****).** Microbiota of patients belonging to Cluster A (*n*=12 at baseline) was driven by *Faecalibacterium* and other *Firmicutes* (unclassified *Ruminococcaceae, Clostridium* XIVa and *Blautia*). Cluster B (*n*=10 at baseline) was enriched in *Bacteroides,* and Cluster C (*n*=4 at baseline) was enriched in *Prevotella* **(****Fig. 3B****).** Cluster A was associated with longer progression-free survival than Cluster B (p=0.0039; **Fig. 3D****)** and, to a lesser extent, longer overall survival (p=0.051; **Fig. 3C****).** Most of the 22 patients from the Bacteroides-driven Cluster B (80%) received subsequent treatments, including anti-PD-1 agents in 50% of the patients while 58% of patients from the Faecalibacterium-driven Cluster A were treated with subsequent therapies, but only 25% of them received anti-PD-1 agents. Cluster A was also associated with a higher proportion of long-term clinical benefit (8/12; 67%) compared to Cluster B (0/10; *p*= 0.001688, Fisher's exact test).

At the phyla level, microbiota of patients prone to develop colitis was enriched in Firmicutes at baseline (Wilcoxon test, *p*=0.009), while high proportions of Bacteroidetes was observed in patients who did not develop colitis (*p*=0.011; **Fig. 4A****).** In competing risk analysis, belonging to Cluster A (driven by *Faecalibacterium* and other Firmicutes) tended to be associated with a shorter colitis-free cumulative incidence compared to Cluster B (driven by *Bacteroides*) (Gray's test; *p*=0.0545, **Fig. 4B****).** Using linear discriminant analysis effect size (LEfSe) analysis, fifteen bacterial OTUs were detected as potential biomarkers either of colitis-free progression or colitis onset during ipilimumab treatment **(****Fig. 4C****).** Five (out of six) OTUs from the Bacteroidetes phylum (such as *B. uniformis, B. vulgatus* and *Parabacteroides distasonis*) were associated with absence of colitis, whereas OTUs associated with subsequent colitis related mostly to the Firmicutes phylum (8/9 OTUs). Several of these potential colitis-predictive baseline OTUs (relatives of *Faecalibacterium prausnitzii* L2-6, *butyrate producing bacterium* L2-21 and *Gemmiger formicilis* ATCC 27749) were also associated with longer overall survival.

### Baseline immune parameters related to microbiota clustering, clinical benefit and ipilimumab-induced colitis.

Twenty-six patients had both immune monitoring and microbiota composition analysis at V₁. Patients from Cluster C could not be included in this analysis due to the low number of patients belonging to this cluster (*n*=4). Since patients who belonged to Cluster A (*n*=12) and Cluster B (*n*=10) had no differences in absolute counts of peripheral-blood CD4⁺ and CD8⁺ T cells (Table S5), the main T cell subpopulations were considered as a percentage. Patients who belonged to the *Faecalibacterium*-driven Cluster A had a low proportion of baseline regulatory T cells (Tregs), and had a significantly lower proportion of baseline α4⁺β7⁺ CD4⁺ (*p*=0.0447) and α4⁺β7⁺ CD8⁺ (p=0.0344) T cells, compared to those who belonged to the *Bacteroides*-driven Cluster B **(****Fig. 5A**).

Patients with long-term benefit tended to have a lower frequency of regulatory T cells (p=0.056) and a lower frequency of α4⁺β7⁺ CD4⁺ (p=0.014) and α4⁺β7⁺ CD8⁺ (*p*=0.0081) T cells, compared to those with poor clinical benefit **(****Fig. 5B****).** Although serum concentrations of several biomarkers of inflammation (IL-6, IL-8, IP-10, MCP-1, TNFα, sCD25) and sCD14 considered as a marker of bacterial invasion (translocation) were measured, none of these were associated with any cluster or clinical benefit at baseline.

Patients who developed ipilimumab-induced colitis tended to have significantly higher absolute numbers of CD4+ T cells (p=0.0529) and had significantly lower levels of IL-6, IL-8 and sCD25 at baseline compared to patients without colitis **(****Fig. 5C****).** In competing risk analysis, patients with high numbers of conventional CD4⁺ T cells had a shorter colitis-free cumulative incidence, compared to those with low numbers of conventional CD4⁺ T cells (p=0.0038). The median percentage of Tregs were lower before introduction of ipilimumab in patients who developed colitis during the course of ipilimumab compared to patients without colitis (2.5% vs 8.4%, *p*=0.0182). However, absolute numbers of Tregs were not significantly different. None of the other CD4⁺ T cell subpopulations at baseline were related to colitis onset.

### The Inducible T-cell COStimulator (ICOS) molecule is significantly up-regulated on CD4⁺ T cells after ipilimumab treatment in patients who belong to Faecalibacterium-driven Cluster A

Since immune parameters were monitored longitudinally, specific changes in immune activation during the course of ipilimumab treatment were assessed in patients from Cluster A *vs* B. During ipilimumab treatment, Inducible T-cell COStimulator (ICOS) was significantly increased on conventional CD4⁺ T and Treg cells in patients who belonged to Cluster A **(Fig. 6A-B),** but not in those who belonged to Cluster B **(Fig. 6A-B).** Given that ICOS is induced on CD4⁺ T cells after IL-2 stimulation, concentration of sCD25, a marker of IL-2 impregnation [29, 30], was surveyed during the course of ipilimumab treatment. Patients who belonged to Cluster A showed a greater increase over time in sCD25 than patients from Cluster B, as demonstrated by a higher V_{2/3} to V₁ ratio compared to patients from Cluster B **(Fig. 6C).**

### III. CONCLUSION

This study shows that a distinct baseline gut microbiota composition is associated with an anti-cancer response and immune-mediated colitis in patients with metastatic melanoma treated with ipilimumab. Colonization by Firmicutes and more specifically by OTUs related to *Faecalibacterium prausnitzii* L2-6, *butyrate producing bacterium* L2-21 and *Gemmiger formicilis* ATCC 27749, is associated with both anti-cancer response and immune-related colitis. There is a higher representation of Bacteroidetes (mostly *Bacteroides* genus) in patients who have a poor anti-cancer response and who remain colitis-free.

In addition, in our dataset, *Bacteroides fragilis* and *Bacteroides thetaiotaomicron* were not abundantly represented at baseline. Overall, these bacterial species were more abundant in patients with poor clinical benefit who remained colitis-free.

We found that the *Faecalibacterium*-driven Cluster A and clinical benefit were associated with low baseline percentage of circulating α4⁺β7⁺ T cells and CD4⁺ Tregs. In addition, high proportions of *Faecalibacterium prausnitzii,* low baseline percentage of circulating CD4⁺ Tregs and low baseline levels of systemic inflammatory proteins, such as IL-6 IL-8 and sCD25, were associated with subsequent colitis.

It has also been observed that patients who belong to the *Faecalibacterium*-driven Cluster A have a higher ICOS induction on CD4⁺ T cells and a higher increase in sCD25.

Taken together, these observations show that gut microbiota composition at baseline can predict which patients will adequately respond to ipilimumab.

### IV. Result of metabolomics studies

From the OTUs population described in the overall cohort, metabolomic profiles have been deduced *in silico* applying the PICRUSt algorithm combined with LEFSe.

A specific set of metabolic pathways (described as KEGG IDs) have been highlighted specifically associated with response to anti CTLA4 (see Table V below) or toxicity to anti CTLA4 (see Table IV below).

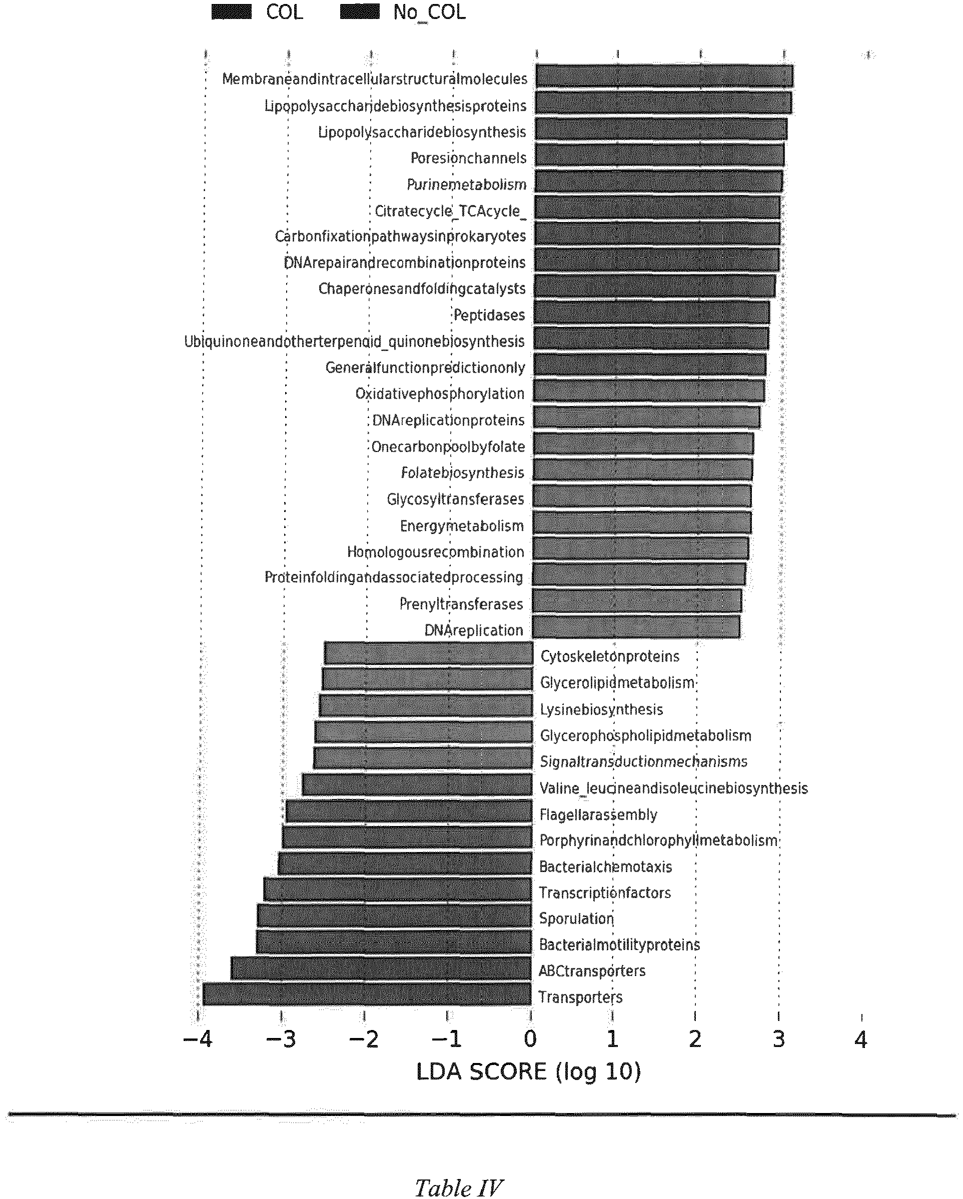

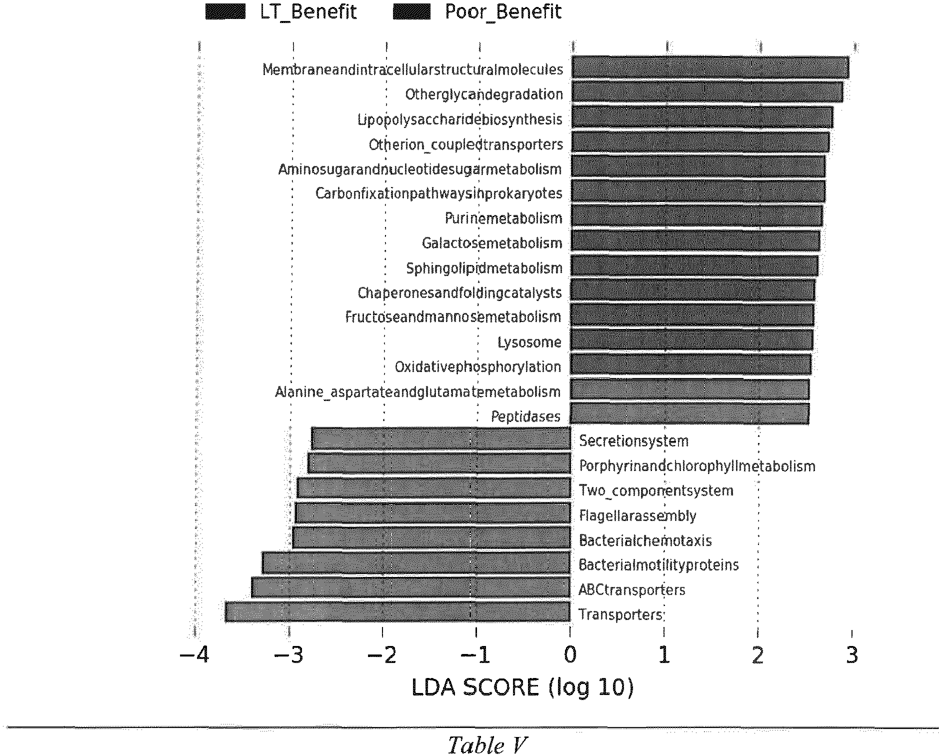

### References

1. Robert C, Thomas L, Bondarenko I et al. Ipilimumab plus dacarbazine for previously untreated metastatic melanoma. N. Engl. J. Med. 2011; 364(26):2517-2526.
2. Hodi FS, O'Day SJ, McDermott DF et al. Improved Survival with Ipilimumab in Patients with Metastatic Melanoma. N. Engl. J. Med. 2010; 363(8):711-723.
3. Schadendorf D, Hodi FS, Robert C et al. Pooled Analysis of Long-Term Survival Data From Phase II and Phase III Trials of Ipilimumab in Unresectable or Metastatic Melanoma. J. Clin. Oncol. 2015; 33(17):1889-1894.
4. Pardoll DM. The blockade of immune checkpoints in cancer immunotherapy. Nat. Rev. Cancer 2012; 12(4):252-264.
5. Postow MA, Callahan MK, Wolchok JD. Immune Checkpoint Blockade in Cancer Therapy. J. Clin. Oncol. 2015; 33(17):1974-1982.
6. Weber JS, Kahler KC, Hauschild A. Management of Immune-Related Adverse Events and Kinetics of Response With Ipilimumab. J. Clin. Oncol. 2012; 30(21):2691-2697.
7. Beck KE, Blansfield JA, Tran KQ et al. Enterocolitis in Patients With Cancer After Antibody Blockade of Cytotoxic T-Lymphocyte-Associated Antigen 4. J. Clin. Oncol. 2006; 24(15):2283-2289.
8. Gupta A, De Felice KM, Loftus EV, Khanna S. Systematic review: colitis associated with anti-CTLA-4 therapy. Aliment. Pharmacol. Ther. 2015; 42(4):406-417.
9. Marthey L, Mateus C, Mussini C et al. Cancer Immunotherapy with Anti-CTLA-4 Monoclonal Antibodies Induces an Inflammatory Bowel Disease. ECCOJC 2016; 10(4):395-401.
10. Abraham C, Cho JH. Inflammatory Bowel Disease. N. Engl. J. Med. 2009; 361(21):2066-2078.
11. Chassaing B, Darfeuille-Michaud A. The Commensal Microbiota and Enteropathogens in the Pathogenesis of Inflammatory Bowel Diseases. Gastroenterology 2011; 140(6):1720-1728.e3.
12. Lepage P, Häsler R, Spehlmann ME et al. Twin Study Indicates Loss of Interaction Between Microbiota and Mucosa of Patients With Ulcerative Colitis. Gastroenterology 2011; 141(1):227-236.
13. Vétizou M, Pitt JM, Daillère R et al. Anticancer immunotherapy by CTLA-4 blockade relies on the gut microbiota. Science 2015; 350(6264):1079-1084.
14. Sivan A, Corrales L, Hubert N et al. Commensal Bifidobacterium promotes antitumor immunity and facilitates anti-PD-L1 efficacy. Science 2015; 350(6264):1084-1089.
15. Wolchok JD, Hoos A, O'Day S et al. Guidelines for the evaluation of immune therapy activity in solid tumors: immune-related response criteria. Clin. Cancer Res. Off. J. Am. Assoc. Cancer Res. 2009; 15(23):7412-7420.
16. Snyder A, Makarov V, Merghoub T et al. Genetic basis for clinical response to CTLA-4 blockade in melanoma. N. Engl. J. Med. 2014; 371(23):2189-2199.
17. Seksik P. Alterations of the dominant faecal bacterial groups in patients with Crohn's disease of the colon. Gut 2003; 52(2):237-242.
18. Chen H, Liakou CI, Kamat A et al. Anti-CTLA-4 therapy results in higher CD4+ICOShi T cell frequency and IFN-gamma levels in both nonmalignant and malignant prostate tissues. Proc. Natl. Acad. Sci. U. S. A. 2009; 106(8):2729-2734.
19. Read S, Greenwald R, Izcue A et al. Blockade of CTLA-4 on CD4+CD25+ regulatory T cells abrogates their function in vivo. J. Immunol. Baltim. Md 1950 2006; 177(7):4376-4383.
20. Lukiw WJ. Bacteroides fragilis Lipopolysaccharide and Inflammatory Signaling in Alzheimer's Disease. Front. Microbiol. 2016; 7:1544.
21. Guigoz Y, Doré J, Schiffrin EJ. The inflammatory status of old age can be nurtured from the intestinal environment. Curr. Opin. Clin. Nutr. Metab. Care 2008; 11(1):13-20.
22. Le Chatelier E, Nielsen T, Qin J et al. Richness of human gut microbiome correlates with metabolic markers. Nature 2013; 500(7464):541-546.
23. Sokol H, Pigneur B, Watterlot L et al. Faecalibacterium prausnitzii is an antiinflammatory commensal bacterium identified by gut microbiota analysis of Crohn disease patients. Proc. Natl. Acad. Sci. U. S. A. 2008; 105(43):16731-16736.
24. Devillard E, McIntosh FM, Duncan SH, Wallace RJ. Metabolism of linoleic acid by human gut bacteria: different routes for biosynthesis of conjugated linoleic acid. J. Bacteriol. 2007; 189(6):2566-2570.
25. Mathewson ND, Jenq R, Mathew AV et al. Gut microbiome-derived metabolites modulate intestinal epithelial cell damage and mitigate graft-versus-host disease. Nat. Immunol. 2016; 17(5):505-513.
26. Smith PM, Howitt MR, Panikov N et al. The microbial metabolites, short-chain fatty acids, regulate colonic Treg cell homeostasis. Science 2013; 341(6145):569-573.
27. Gray RJ. A Class of $K$-Sample Tests for Comparing the Cumulative Incidence of a Competing Risk. Ann Stat. 1988; 16(3):1141-1154.
28. Lin G, So Y, Johnston G. Analysing survival data with competing risks using SAS* software. Proc. SAS Glob. Forum 2012 Conf. Cary NC SAS Inst. Inc 2012.
29. Riley JL, Blair PJ, Musser JT et al. ICOS Costimulation Requires IL-2 and Can Be Prevented by CTLA-4 Engagement. J. Immunol. 2001; 166(8):4943-4948.
30. Lotze MT, Custer MC, Sharrow SO et al. In vivo administration of purified human interleukin-2 to patients with cancer: development of interleukin-2 receptor positive cells and circulating soluble interleukin-2 receptors following interleukin-2 administration. Cancer Res. 1987; 47(8):2188-2195.
31. Dubin K, Callahan MK, Ren B et al. Intestinal microbiome analyses identify melanoma patients at risk for checkpoint-blockade-induced colitis. Nat. Commun. 2016; 7:10391.
32. Maynard CL, Elson CO, Hatton RD, Weaver CT. Reciprocal interactions of the intestinal microbiota and immune system. Nature 2012; 489(7415):231-241.
33. Cebula A, Seweryn M, Rempala GA et al. Thymus-derived regulatory T cells contribute to tolerance to commensal microbiota. Nature 2013; 497(7448):258-262.
34. Png CW, Linden SK, Gilshenan KS et al. Mucolytic bacteria with increased prevalence in IBD mucosa augment in vitro utilization of mucin by other bacteria. Am. J. Gastroenterol. 2010; 105(11):2420-2428.
35. Hedin CR, McCarthy NE, Louis P et al. Altered intestinal microbiota and blood T cell phenotype are shared by patients with Crohn's disease and their unaffected siblings. Gut 2014; 63(10):1578-1586.
36. Romano E, Kusio-Kobialka M, Foukas PG et al. Ipilimumab-dependent cell-mediated cytotoxicity of regulatory T cells ex vivo by nonclassical monocytes in melanoma patients. Proc. Natl. Acad. Sci. U. S. A. 2015; 112(19):6140-6145.
37. Fu T, He Q, Sharma P. The ICOS/ICOSL Pathway Is Required for Optimal Antitumor Responses Mediated by Anti-CTLA-4 Therapy. Cancer Res. 2011; 71(16):5445-5454.
38. Liakou CI, Kamat A, Tang DN et al. CTLA-4 blockade increases IFN -producing CD4+ICOShi cells to shift the ratio of effector to regulatory T cells in cancer patients. Proc. Natl. Acad. Sci. 2008; 105(39):14987-14992.
39. Sim GC, Martin-Orozco N, Jin L et al. IL-2 therapy promotes suppressive ICOS+ Treg expansion in melanoma patients. J. Clin. Invest. 2014; 124(1):99-110.
40. Klatzmann D, Abbas AK. The promise of low-dose interleukin-2 therapy for autoimmune and inflammatory diseases. Nat. Rev. Immunol. 2015; 15(5):283-294.
41. Chaput N, Flament C, Locher C et al. Phase I clinical trial combining imatinib mesylate and IL-2: HLA-DR(+) NK cell levels correlate with disease outcome. Oncoimmunology 2013; 2(2):e23080.
42. Yu A, Snowhite I, Vendrame F et al. Selective IL-2 responsiveness of regulatory T cells through multiple intrinsic mechanisms supports the use of low-dose IL-2 therapy in type 1 diabetes. Diabetes 2015; 64(6):2172-2183.
43. Hodi FS, Chesney J, Pavlick AC et al. Combined nivolumab and ipilimumab versus ipilimumab alone in patients with advanced melanoma: 2-year overall survival outcomes in a multicentre, randomised, controlled, phase 2 trial. Lancet Oncol. 2016; 17(11):1558-1568.
44. Larkin J, Hodi FS, Wolchok JD. Combined Nivolumab and Ipilimumab or Monotherapy in Untreated Melanoma. N. Engl. J. Med. 2015; 373(13):1270-1271.

## Claims

1. Method for predicting the response of an individual to an anticancer treatment with anti-CTL4 molecules comprising the steps of:
(i) determining gut microbial OTU in a fecal sample of said individual;
(ii) determining a relative abundance of at least one OTU comprising a nucleotide fragment of sequence having at least 97%, preferably 98%, 99% or 100%, identity with a nucleic sequence selected in the group consisting of: denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7, denovo4054 of SEQ. ID. N°8, denovo3816 of SEQ. ID. N°9, denovo3657 of SEQ. ID. N°10, denovo5800 of SEQ. ID. N°11 and denovo5178 of SEQ. ID. N°12; preferably, determining a relative abundance of at least one OTU comprising a nucleotide fragment of sequence having at least 97%, preferably 98%, 99% or 100%, identity with a nucleic sequence selected in the group consisting of: denovo3066 of SEQ. ID. N°1, denovo991 of SEQ. ID. N°2, denovo3795 of SEQ. ID. N°5, denovo4787 of SEQ. ID. N°6, denovo4054 of SEQ. ID. N°8, denovo3816 of SEQ. ID. N°9, denovo3657 of SEQ. ID. N°10 and denovo5178 of SEQ. ID. N°12;
wherein individual having a gut microbiote enriched in at least one OTU selected in the group consisting of denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7 and denovo5178 of SEQ. ID. N°12 and/or depleted in at least one OTU selected in the group consisting of denovo4054 of SEQ. ID. N°8, denovo3816 of SEQ. ID. N°9, denovo3657 of SEQ. ID. N°10 and denovo5800 of SEQ. ID. N°11 is a good responder to said anticancer treatment.

2. Method for predicting the response of an individual to an anticancer treatment with anti-CTL4 molecules according to claim 1, wherein step (ii) consists in determining the relative abundance of at least two, three, four, five or six sequences having at least 97%, preferably 98%, 99% or 100%, identity with the nucleic sequence of OTUs selected in the group consisting of: denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7, denovo4054 of SEQ. ID. N°8, denovo3816 of SEQ. ID. N°9, denovo3657 of SEQ. ID. N°10, denovo5800 of SEQ. ID. N°11, denovo5178 of SEQ. ID. N°12, denovo3073 of SEQ. ID. N°13, denovo3428 of SEQ. ID. N°14, denovo892 of SEQ. ID. N°15 and denovo2582 of SEQ. ID. N°16

3. Method for predicting the response of an individual to an anticancer treatment with anti-CTL4 molecules according to claim 2, wherein the following relative abundance of said combinations of OTUs in the gut microbiote of an individual means that said individual is a good responder to said anticancer treatment:
| Combination | enrichment | depletion |
|---|---|---|
| A | SEQ. ID. N°: 7, 12 | SEQ. ID. N°: 8, 9, 10, 11 |
| B | SEQ. ID. N°: 12 | SEQ. ID. N°: 8, 9, 10, 11 |
| C | SEQ. ID. N°:1, 6, 7 | SEQ. ID. N°: 8, 9, 10 |
| D | SEQ. ID. N°: 1, 7 | SEQ. ID. N°: 8, 9, 10 |
| E | SEQ. ID. N°: 1, 6 | SEQ. ID. N°: 9, 10 |
| F | SEQ. ID. N°: 1, 7 | SEQ. ID. N°: 9, 10 |
| G | SEQ. ID. N°: 1 | SEQ. ID. N°: 8, 9, 10 |
| H | SEQ. ID. N°: 1 | SEQ. ID. N°: 9, 10 |
| I | SEQ. ID. N°: 1 | SEQ. ID. N°: 9, 10, 13 |
| J | SEQ. ID. N°: 1 | SEQ. ID. N°: 9, 10, 14 |
| K | SEQ. ID. N°: 1 | SEQ. ID. N°: 9, 10, 15 |
| L | SEQ. ID. N°: 1, 16 | SEQ. ID. N°: 9, 10 |

4. Method for predicting the response of an individual to an anticancer treatment with anti-CTL4 molecules comprising the steps of:
(i) determining gut microbial OTU in a fecal sample of said individual;
(ii) determining the presence or the absence of at least one OTU comprising a nucleotide fragment of sequence having at least 97%, preferably 98%, 99% or 100%, identity with a nucleic sequence selected in the group consisting of: denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7, denovo4054 of SEQ. ID. N°8, denovo3816 of SEQ. ID. N°9, denovo3657 of SEQ. ID. N°10, denovo5800 of SEQ. ID. N°11 and denovo5178 of SEQ. ID. N°12, by applying a detection threshold of 0,2% of the total number of sequences (per sample) to define an OTU's presence or absence, or applying quantitative PCR detection with specific OTU or bacterial isolate targeting probes;
wherein individual having a gut microbiote showing the presence of at least one OTU selected in the group consisting of denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7 and denovo5178 of SEQ. ID. N°12 and/or showing the absence of at least one OTU selected in the group consisting of denovo4054 of SEQ. ID. N°8, denovo3816 of SEQ. ID. N°9, denovo3657 of SEQ. ID. N°10 and denovo5800 of SEQ. ID. N°11 is a good responder to said anticancer treatment.

5. Method for predicting the response of an individual to an anticancer treatment with anti-CTL4 molecules according to claim 4, wherein step (ii) consists in determining the presence or the absence of at least two, three, four, five or six sequences having at least 97%, preferably 98%, 99% or 100%, identity with the nucleic sequence of OTUs selected in the group consisting of: denovo3066 of SEQ. ID. N°1, denovo991 of SEQ. ID. N°2, denovo4666 of SEQ. ID. N°3, denovo5905 of SEQ. ID. N°4, denovo3795 of SEQ. ID. N°5, denovo4787 of SEQ. ID. N°6, denovo453 of SEQ. ID. N°7, denovo4054 of SEQ. ID. N°8, denovo3816 of SEQ. ID. N°9, denovo3657 of SEQ. ID. N°10, denovo5800 of SEQ. ID. N°11, denovo5178 of SEQ. ID. N°12, denovo3073 of SEQ. ID. N°13, denovo3428 of SEQ. ID. N°14, denovo892 of SEQ. ID. N°15 and denovo2582 of SEQ. ID. N°16.

6. Method for predicting the response of an individual to an anticancer treatment with anti-CTL4 molecules according to claim 5, wherein the following results observed for said combinations of OTUs in the gut microbiote of an individual means that said individual is a good responder to said anticancer treatment:
| Combination | presence | absence |
|---|---|---|
| A | SEQ. ID. N°: 7, 12 | SEQ. ID. N°: 8, 9, 10, 11 |
| B | SEQ. ID. N°: 12 | SEQ. ID. N°: 8, 9, 10, 11 |
| C | SEQ. ID. N°:1, 6, 7 | SEQ. ID. N°: 8, 9, 10 |
| D | SEQ. ID. N°: 1, 7 | SEQ. ID. N°: 8, 9, 10 |
| E | SEQ. ID. N°: 1,6 | SEQ. ID. N°: 9, 10 |
| F | SEQ. ID. N°: 1, 7 | SEQ. ID. N°: 9, 10 |
| G | SEQ. ID. N°: 1 | SEQ. ID. N°: 8, 9, 10 |
| H | SEQ. ID. N°: 1 | SEQ. ID. N°: 9, 10 |
| I | SEQ. ID. N°: 1 | SEQ. ID. N°: 9, 10, 13 |
| J | SEQ. ID. N°: 1 | SEQ. ID. N°: 9, 10, 14 |
| K | SEQ. ID. N°: 1 | SEQ. ID. N°: 9, 10, 15 |
| L | SEQ. ID. N°: 1, 16 | SEQ. ID. N°: 9, 10 |

7. Method for predicting the response of an individual to an anticancer treatment with anti-CTL4 molecules comprising the steps of:
(i) culturing a feces sample on appropriate culture medium and in appropriate culture conditions;
(ii) detecting the relative abundance and/or the presence or absence of at least one of the bacterial isolate/species affiliated to the OTU of SEQ. ID. N°1 to 12, wherein enrichment or presence in an individual's gut microbiote of bacterial isolate/species selected in the group consisting of *Parabacteroides distasonis, uncultured bacterium NA48 AY975552 (Clostridium XIVa*/ *Fusicatenibacter saccharivorans*), *butyrate producing bacterium SS2 1 AY305319 (Anaerostipes hadrus), uncultured bacterium C3 13 GQ896957 (Ruminococcus*/*Oscillibacter valericigenes), Faecalibacterium prausnitzii, Gemmiger formicilis, uncultured bacterium RL246 aai74e12 DQ793623 (Unclassified Clostridiales*/*Faecalibacterium sp. canine oral taxon* 147) and *butyrate producing bacterium L2 21 AJ270477* / *Eubacterium rectale* and/or the depletion or absence in said individual's gut microbiote of bacterial isolate/species selected in the group consisting of *Bacteroides ovatus, Faecalibacterium prausnitzii, Clostridium sp. XB90* / *Eisenbergiella massiliensis and uncultured bacterium REC1M 21 AY343160 (Oscillibacter sp. Marseille-P2778)* means that said individual is a good responder to said anticancer treatment.

8. Method for predicting the response of an individual to an anticancer treatment with anti-CTL4 molecules comprising the steps of:
(i) determining gut microbial OTU in a fecal sample of said individual;
(ii) determining the relative abundance of at least one OTU comprising a nucleic acid fragment of 300 to 1500 pb having a sequence of at least 97%, preferably 98%, 99% or 100%, identity with a nucleic acid fragment sequence of a 16S rRNA selected in the group consisting of: SEQ. ID. N°16, SEQ. ID. N°17, SEQ. ID. N°18, SEQ. ID. N°19, SEQ. ID. N°20, SEQ. ID. N°21, SEQ. ID. N°22, SEQ. ID. N°23, SEQ. ID. N°24, SEQ. ID. N°25, SEQ. ID. N°26, SEQ. ID. N°27 and SEQ. ID. N°28.

9. Method for predicting the response of an individual to an anticancer treatment with anti-CTL4 molecules comprising the step of determining the presence or the absence of at least one metabolite (described as KEGG ID) selected in the group consisting of:
Membrane and intracellular structural molecules
Other glycan degradation
Lipopolysaccharide biosynthesis
Other ion_coupled transporters
Aminosugar and nucleotidesugar metabolism
Carbon fixation pathways in prokaryotes
Purine metabolism
Galactose metabolism
Sphingolipid metabolism
Chaperones and folding catalysts
Fructose and mannose metabolism
Lysosome
Oxidative phosphorylation
Alanine_aspartate and glutamate metabolism
Peptidases
Secretion system
Porphyrin and chlorophyll metabolism
Two_component system
Flagellar assembly
Bacterial chemotaxis
Bacterial motility proteins
ABC transporters
Transporters
in a biological sample of said individual, wherein the presence of at least one metabolite selected in the group consisting of:
Secretion system
Porphyrin and chlorophyll metabolism
Two_component system
Flagellar assembly
Bacterial chemotaxis
Bacterial motility proteins
ABC transporters
Transporters
is associated with a good response to said anti-cancer treatment and/or the presence of at least one metabolite selected in the group consisting of:
Membrane and intracellular structural molecules
Other glycan degradation
Lipopolysaccharide biosynthesis
Other ion_coupled transporters
Aminosugar and nucleotidesugar metabolism
Carbon fixation pathways in prokaryotes
Purine metabolism
Galactose metabolism
Sphingolipid metabolism
Chaperones and folding catalysts
Fructose and mannose metabolism
Lysosome
Oxidative phosphorylation
Alanine_aspartate and glutamate metabolism
Peptidases
is associated with a poor response to said anti-cancer treatment.

10. Method according to anyone of claim 1 to 9 wherein said individual is a patient with metastatic melanoma and/or said anti-CTL4 molecule is ipilimumab.
